# EUROPEAN PATENT SPECIFICATION

(11) **EP 2 398 917 B1**
(45) Date of publication and mention of the grant of the patent: **05.10.2016**
(21) Application number: 10743317.9
(22) Date of filing: 17.02.2010
(51) Int. Cl.: C12Q 1/68

(54) **ASSAY FOR DETERMINING EPIGENETIC PROFILES OF MARKERS OF FRAGILE X ALLELES**
TEST ZUR BESTIMMUNG DER EPIGENETISCHEN PROFILE VON MARKERN FRAGILER X-ALLELE
DOSAGE PERMETTANT DE DÉTERMINER LES PROFILS ÉPIGÉNÉTIQUES DES MARQUEURS D'ALLÈLES DE L'X FRAGILE

(30) Priority: 17.02.2009 AU 2009900668; 11.03.2009 AU 2009901041
(43) Date of publication of application: 28.12.2011
(73) Proprietor: Murdoch Childrens Research Institute, Parkville, VIC 3052 (AU)
(72) Inventor: GODLER, David Eugeny, Ormond, Victoria 3204 (AU)
(74) Representative: Jones, Elizabeth Louise
(86) International application number: PCT/AU2010/000169
(87) International publication number: WO 2010/094061

(56) References cited:
- WO-A1-92/20825
- WO-A1-2009/045467
- WO-A2-2008/045136
- US-A- 6 143 504
- US-A- 6 150 100
- SABINE SCHWEMMLE ET AL: "Characterization of FMR1 Promoter Elements by In Vivo- Footprinting Analysis", AM. J. HUM. GENET., vol. 60, no. 6, 1 June 1997 (1997-06-01), pages 1354-1362, XP55029389,
- PIETROBONO R ET AL: "Quantitative analysis of DNA demethylation and transcriptional reactivation of the FMR1 gene in fragile X cells treated with 5-azadeoxycytidine", NUCLEIC ACIDS RESEARCH SPECIAL PUBLICATION, OXFORD UNIVERSITY PRESS, SURREY, GB, vol. 30, no. 14, 1 July 2002 (2002-07-01), pages 3278-3285, XP003016360, ISSN: 0305-1048, DOI: 10.1093/NAR/GKF434
- GHELDOF NELE ET AL: "The active FMR1 promoter is associated with a large domain of altered chromatin conformation with embedded local histone modifications", PROCEEDINGS OF THE NATIONAL ACADEMY OF SCIENCES, NATIONAL ACADEMY OF SCIENCES, US, vol. 103, no. 33, 15 August 2006 (2006-08-15), pages 12463-12468, XP002598281, ISSN: 0027-8424, DOI: 10.1073/PNAS.0605343103
- WEINHAUSEL ANDREAS ET AL: "Evaluation of the fragile X (FRAXA) syndrome with methylation-sensitive PCR", HUMAN GENETICS, SPRINGER, BERLIN, DE, vol. 108, no. 6, 1 June 2001 (2001-06-01), pages 450-458, XP002195971, ISSN: 0340-6717, DOI: 10.1007/S004390100519
- ZHOU Y ET AL: "Simplified molecular diagnosis of fragile X syndrome by fluorescent methylation-specific PCR and GeneScan analysis", CLINICAL CHEMISTRY, AMERICAN ASSOCIATION FOR CLINICAL CHEMISTRY, WASHINGTON, DC, vol. 52, no. 8, 1 August 2006 (2006-08-01) , pages 1492-1500, XP002592249, ISSN: 0009-9147, DOI: 10.1373/CLINCHEM.2006.068593 [retrieved on 2006-06-22]
- OOSTRA B A ET AL: "Diagnostic tests for fragile X syndrome.", EXPERT REVIEW OF MOLECULAR DIAGNOSTICS JUL 2001 LNKD- PUBMED:11901818, vol. 1, no. 2, July 2001 (2001-07), pages 226-232, XP009160122, ISSN: 1473-7159
- TASSONE F ET AL: "Expression of the FMR1 gene.", CYTOGENETIC AND GENOME RESEARCH 2003 LNKD- PUBMED:14526172, vol. 100, no. 1-4, 2003, pages 124-128, XP009160068, ISSN: 1424-859X
- ELIZABETH BERRY-KRAVIS ET AL: "Fragile X-associated tremor/ataxia syndrome: Clinical features, genetics, and testing guidelines", MOVEMENT DISORDERS, vol. 22, no. 14, 31 October 2007 (2007-10-31), pages 2018-2030, XP55029531, ISSN: 0885-3185, DOI: 10.1002/mds.21493
- K. CORNISH ET AL: "The fragile X continuum: new advances and perspectives", JOURNAL OF INTELLECTUAL DISABILITY RESEARCH, vol. 52, no. 6, 1 June 2008 (2008-06-01), pages 469-482, XP55029530, ISSN: 0964-2633, DOI: 10.1111/j.1365-2788.2008.01056.x
- STEPHEN T. WARREN: "The Epigenetics of Fragile X Syndrome", CELL STEM CELL, vol. 1, no. 5, 1 November 2007 (2007-11-01), pages 488-489, XP55029388, ISSN: 1934-5909, DOI: 10.1016/j.stem.2007.10.017
- DAHL, C. ET AL.: 'A Homogeneous Assay for Analysis of FMR1 Promoter Methylation in Patients with Fragile X Syndrome' CLINICAL CHEMISTRY vol. 53, 2007, pages 790 - 793, XP055029491
- OBERLE, I. ET AL.: 'Instability of a 550-Base Pair DNA Segment and Abnormal Methylation in Fragile X Syndrome' SCIENCE vol. 252, 1991, pages 1097 - 1102, XP055029492
- IRIZARRY, R.A. ET AL.: 'The human colon cancer methylome shows similar hypo- and hypermethylation at conserved tissue-specific CpG island shores' NATURE GENETICS vol. 41, 2009, pages 178 - 186, XP055029485
- KHALIFA, M.M. ET AL.: 'Methylation Status of Genes Flanking the Fragile Site in Males with the Fragile-X Syndrome: A Test of the Imprinting Hypothesis' AMERICAN JOURNAL OF HUMAN GENETICS vol. 46, 1990, pages 744 - 753, XP009160108
- GODLER, D.E. ET AL.: 'Methylation of novel markers of fragile X alleles is inversely correlated with FMRP expression and FMRl activation ratio' HUMAN MOLECULAR GENETICS vol. 19, 2010, pages 1618 - 1632, XP055029010
- "FMR1 Antisense RNA 1", INTERNET CITATION, XP007923067, [retrieved on 2015-03-25]

## Description

### FIELD

The present invention relates generally to an assay for the determination of epigenetic profiles, particularly epigenetic profiles associated with a pathological condition. Even more particularly, the present invention provides an assay to detect epigenetic profiles within the Fragile X Mental Retardation (FMR) genetic locus indicative of a pathoneurological condition such as pathoneurodevelopmental and pathoneurodegenerative conditions. The epigenetic profiles can also identify potential non-neurological conditions. Kits also form part of the present invention as do computer programs to monitor changes in epigenetic patterns and methods for screening for agents which modulate epigenetic modification.

### BACKGROUND

Bibliographic details of the publications referred to in this specification are also collected at the end of the description.

Reference to any prior art in this specification is not, and should not be taken as, an acknowledgment or any form of suggestion that this prior art forms part of the common general knowledge in any country.

The Fragile X Mental Retardation genetic locus ("FMR genetic locus") includes the FMR1 gene which is composed of 17 exons, spanning 38Kb, and encodes Fragile X Mental Retardation Protein (FMRP), essential for normal neurodevelopment *(*Verkerk et al, Cell 65(5):905-914, 1991; Terracciano et al, Am JMed Genet C Semin Med Genet 137C(1):32-37, 2005). A CGG repeat segment is located within the 5' untranslated region (UTR) of the gene. Its normal range is < 40 repeats. When expanded, these repeats have been implicated in a number of pathologies, including the Fragile X syndrome (FXS), Fragile X-associated Tremor Ataxia Syndrome (FXTAS) and Fragile X-associated primary ovarian insufficiency (FXPOI; formerly referred to as Premature Ovarian Failure [POF]). FXS is neurodevelopmental in nature with a frequency of 1/1400 males and 1/800 females, associated with a Fragile site at the Xq27.3 locus (Jin and Warren, Hum. Mol. Genet 9(6):901-908, 2000).

This syndrome is caused by CGG expansion to "full mutation" (FM) which comprises >200 repeats, leading to a gross deficit of FMRP and subsequent synaptic abnormalities (Pieretti et al, Cell 66(4):817-822, 1991; Irwin et al, Cereb Cortex 10(10):1038-1044, 2000). The FXS clinical phenotype ranges from learning disabilities to severe mental retardation and can be accompanied by a variety of physical and behavioral characteristics. FXTAS is prevalent in ∼30% of premutation individuals (PM), comprising - 55 to 199 repeats (Nolin et al, Am JHum Genet 72(2):454-464, 2003) and is a progressive neurodegenerative late-onset disorder with a frequency of 1/3000 males in the general population (Jacquemont et al, Am JMent Retard 109(2):154-164, 2004), manifesting as tremor, imbalance and distinct MRI and histological changes (Hagerman et al, Neurology 57(1):127-130, 2001; Jacquemont et al, JMed Genet 42(2):e14, 2005; Loesch et al, Clin Genet 67(5):412-417, 2005). It is often associated with 'toxicity' of elevated FMR1 mRNA, which has been linked to the intranuclear inclusions and cell death observed during neurodegeneration (Jin et al, Neuron 39(5):739-747, 2003).

US6143504 discloses a method for diagnosis of FXS in males using specific primers to amplify the FMR1 gene promoter and detect its methylation state.

FXTAS can occur in females carrying PM, but with much lower frequency as can be expected from X-linked inheritance. The intermediate or Gray Zone (GZ) alleles comprising 41 to 54 repeats (Bodega et al, Hum Reprod 21(4):952-957, 2006) are the most common form of the expansion, 1 in 30 males and 1 in 15 females. As with PM alleles, increased levels of FMR1 mRNA have been reported in the GZ individuals, proportional to the size of CGG expansion (Kenneson et al, Hum Mol Genet 10(14):14491454, 2001; Mitchell et al, Clin Genet 67(1):38-46, 2005; Loesch et al, J Med Genet 44(3):200-204, 2007). Female carriers of both PM and GZ alleles types have an increased risk of developing POF (Allingham-Hawkins et al, Am J Med Genet 83(4):322-325, 1999; Sullivan et al, Hum Reprod 20(2):402-412, 2005) which has incidence of approximately 1% in the general population, and often unknown etiology (Coulam, Fertil Steril 38(6):645-655, 1982).

Expansion related abnormalities in FMR1 are involved in pathologies with a wide spectrum of patho-mechanisms all pointing to involvement of multiple factors at the Xq27.3 locus in addition to FMR1. A number of antisense transcripts have been described embedded within the FMR1 sequence, ASFMR1 (Ladd et al, Hum Mol Genet 16(24):3174-3187, 2007) and FMR4 (Khalil et al, PLoS ONE 3(1):e1486, 2008). The ASFMR1 and FMR4 transcripts have been suggested to share the bi-directional promoter with FMR1, which is heavily regulated by the state of the surrounding chromatin environment (Pietrobono et al, Nucleic Acids Res 30(14):3278-3285, 2002; Chiurazzi et al, Hum Mol Genet 7(1):109113, 1998).

Transcription of ASFMR1 is also regulated by another promoter located in the exon 2 of FMR1, with the resulting transcript spanning the CGG repeat in the antisense direction (Ladd *et al,* 2007, *supra*), and an open reading frame (ORF) with the CGG encoding a polyproline peptide (Ladd *et al,* 2007, *supra*). FMR4, however, is a long noncoding RNA, involved in regulation of apoptosis (Khalil *et al,* 2008, *supra*).

The length of the CGG repeat has been reported to affect transcription of all three genes FMR1, FMR4 and ASFMR1 (Ladd *et al,* 2007, *supra*; Khalil *et al,* 2008, *supra*). However, although it is well documented that FMR1 transcription is promoter methylation dependent, linked to the CGG expansion size, the relationship between FMR4 and ASFMR1 transcription and methylation remains elusive.

One of the current problems is in the diagnosis of subjects with FM in the FMR genetic locus. Diagnostic assays targeting only the CGG expansion have hitherto been inconclusive. Therefore, currently Southern DNA analysis, which is expensive and time consuming, is used as a gold standard assay for diagnosis in many laboratories.

It is apparent that DNA methylation and other epigenetic modifications play a role in the regulation of gene expression in higher organisms. The importance of epigenetic modification has been highlighted by its involvement in several human diseases. Methylation, for example, of cytosine at the 5' position is the only known methylation modification of genomic DNA. In particular, methylation of CpG islands within regulatory regions of the genome appears to be highly tissue specific. It is now apparent that methylation of cytosines distal to the islands is also important. These regions are called "shores" or "island shores" (Irizarry et al, Nature Genetics 41(2):178-186, 2009). Epigenetic modifications include histone modification, changes in acetylation, methylation, obiquitylation, phosphorylation, sumoylation, activation or deactivation, chromatin altered transcription factor levels and the like.

Despite the availability of a range of methylation assays (see, for example, Rein et al, Nucleic Acids Res. 26:2255, 1998 in relation to methylation assays), selection of regions to amplify and screen is an important aspect of determining an epigenetic profile characteristic of a disease condition. There is a need to identify these crucial regions in the FMR genetic locus involved in regulating expression of the FMR genetic locus and to associate an epigenetic profile to pathological conditions involving this locus. The available range of methylation assays for analysis of the FMR locus is limited to male samples. Thus, there is also a need to develop better assays which can diagnose or identify the FM genotype and methylation or other epigenetic status of the FMR genetic locus in both males and females.

### SUMMARY

Throughout this specification, unless the context requires otherwise, the word "comprise" or variations such as "comprises" or "comprising", will be understood to imply the inclusion of a stated element or integer or group of elements or integers but not the exclusion of any element or integer or group of elements or integers.

Nucleotide and amino acid sequences are referred to by a sequence identifier number (SEQ ID NO:). The SEQ ID NOs: correspond numerically to the sequence identifiers <400>1 (SEQ ID NO:1), <400>2 (SEQ ID NO:2), etc. A summary of sequence identifiers is given in Table 1.

The present invention is predicated in part on the identification of selected regions of the FMR genetic locus, the extent of epigenetic modification of which, is an indicator of a pathological condition associated with the FMR1, FMR4 and ASFMR1 genes. Such conditions include FXS, FXTAS, FXPOI, autism, mental retardation. Other conditions also contemplated herein include Klinefelter's syndrome, Turner's syndrome and a modified X-chromosome. Hence, disclosed herein is an assay to detect an epigenetic profile indicative of a pathological condition associated with the FMR genetic locus (which includes the FMR1, FMR4 and ASFMR1 genes). By "epigenetic modification" includes Epigenetic modifications include histone modification, changes in acetylation, methylation, obiquitylation, phosphorylation, sumoylation, activation or deactivation, chromatin altered transcription factor levels and the like. Methods of the invention requires assessment of the extent of methylation. In a particular embodiment, epigenetic modification includes the methylation state of CpG and CpNpG sites within the FMR genetic locus.

Furthermore, the epigenetic profile is also informative as to the spectrum of disease conditions associated with the FMR genetic locus such as if the subject is normal or has a PM, GZ or FM pathology and/or whether the epigenetic change and/or CGG expansion is heterozygous or homozygous at the FMR allele.

Accordingly, one aspect of the present invention provides an *in vitro* method for identifying in the genome of a mammalian cell including a human cell, a pathological condition associated with the FMR1, FMR4 and/or ASFMR1 genes, said method comprising extracting genomic DNA from the cell and subjecting the DNA to an amplification reaction using primers selective of a region of the FMR genetic locus selected from:
i. Fragile X-related Epigenetic Element 1 (FREE 1) comprising the nucleotide sequence set forth in SEQ ID NO: 16 or a homolog thereof defined by having at least 80% nucleotide sequence identity to SEQ ID NO: 16 or which hybridizes to SEQ ID NO: 16 or its complementary form under medium stringency conditions; and
ii. Fragile X-related Epigenetic Element 2 (FREE2) comprising the nucleotide sequence set forth in SEQ ID NO: 17 or a homolog thereof defined by having at least 80% nucleotide sequence identity to SEQ ID NO: 17 or which hybridizes to SEQ ID NO: 17 or its complementary form under medium stringency conditions,
   and subjecting the amplified DNA to a methylation assay to determine the extent of methylation of the DNA wherein a change in the extent of methylation is indicative of the presence of the pathological condition or propensity to develop same.

Reference to the "FMR genetic locus" includes the FMR1, FMR4 and ASFMR1 genes and corresponds to Xq27.3. The FMR genetic locus comprises a promoter region, a (CGG)n region proximal to the promoter and exonic and intronic region of the FMR1, FMR4 and ASFMR1 genes. In particular, the region downstream of the (CGG)ₙ portion of the FMR1 promoter may comprise an intronic region upstream of exon 1. More particularly, the region comprises a site spanning the intronic region upstream of exon 1 to all or a portion of exon 2 of the FMR genetic locus. In accordance with the invention, the region is Fragile X-related Epigenetic Element 1 (FREE1) as defined by SEQ ID NO:16 or a homolog thereof or a portion or fragment thereof defined by having at least 80% nucleotide sequence identity to SEQ ID NO:16 or which hybridizes to SEQ ID NO:16 or its complementary form under medium stringency conditions; or is Fragile X-related Epigenetic Element 2 (FREE2) as defined by SEQ ID NO:17 or a homolog thereof or a portion or fragment thereof defined by having at least 80% nucleotide sequence identity to SEQ ID NO:17 or which hybridizes to SEQ ID NO:17 or its complementary form under medium stringency conditions. The nucleotide sequence of the FMR1 gene is set forth in SEQ ID NO:18. Further disclosed is amplifying all or part of the (CGG)ₙ expansion and detecting the extent of epigenetic change therein. In a particular embodiment, the epigenetic modification is methylation.

Accordingly, another aspect of the present invention contemplates an *in vitro* method for identifying a pathological condition associated with the FMR1, FMR4 and/or ASFMR1 genes in a mammalian subject including a human, the method comprising screening a cell from said subject for a change relative to a control in the extent of methylation within a region of the FMR genetic locus selected from:
i. Fragile X-related Epigenetic Element 1 (FREE1) comprising the nucleotide sequence set forth in SEQ ID NO:16 or a homolog thereof defined by having at least 80% nucleotide sequence identity to SEQ ID NO:16 or which hybridizes to SEQ ID NO:16 or its complementary form under medium stringency conditions ; and
ii. Fragile X-related Epigenetic Element 2 (FREE2) comprising the nucleotide sequence set forth in SEQ ID NO:17 or a homolog thereof defined by having at least 80% nucleotide sequence identity to SEQ ID NO:17 or which hybridizes to SEQ ID NO:17 or its complementary form under medium stringency conditions,
   wherein a change in extent of methylation relative to a control is indicative of the presence of the pathological condition or a propensity to develop same.

Hence, the present invention provides a method for detecting epigenetic change which is the extent of methylation, in the FMR genetic locus associated with a spectrum of neurodegenerative or neurodevelopmental pathologies such as Fragile X-related conditions such as FXS, FXTAS, autism, mental retardation, Klinefelter's syndrome, Turner's syndrome and a modified X-chromosome. Non-neurological disorders include FXPOI.

A "modified" X-chromosome includes an inactivated X-chromosome or an X-chromosome having a skewed X- inactivation, or inversion, insertion, deletion, duplication or is a hybrid.

The epigenetic profile is determined in the genome of a cell of a subject. Any cell may be tested such as a cell from a post-natal or pre-natal human or embryo. More particularly, the cell is a cultured or uncultured Chorionic Villi Sample (CVS) cell, a lymphoblast cell, a blood cell, a buccal cell, an amniocyte or an EBV transformed lymphoblast cell line.

In a particular embodiment of the present invention, the present mechanism is methylation of CpG and/or CpNpG sites. Methylation is determined by a range of assays including bisulfite MALDI-TOF methylation assay. In an alternative embodiment, methylation is determined by use of methylation sensitive PCR, methylation specific melting curve analysis (MS-MCA) or high resolution melting (MS-HRM); quantification of methylation by MALDI-TOF MS; methylation specific MLPA; methylated-DNA precipitation and methylation-sensitive restriction enzymes (COMPARE-MS); or methylation sensitive oligonucleotide microarray; or antibodies. Other methods include NEXT generation (GEN) and DEEP sequencing or pyrosequencing. However, any assay of methylation status may be employed.

The present invention further provides a method for screening for an agent which modulates methylation of an FMR genetic locus in a mammalian cell including a human cell, the method comprising screening for a change relative to a control in the extent of methylation within a region selected from:
i. Fragile X-related Epigenetic Element 1 (FREE1) comprising the nucleotide sequence set forth in SEQ ID NO:16 or a homolog thereof defined by having at least 80% nucleotide sequence identity to SEQ ID NO:16 or which hybridizes to SEQ ID NO:16 or its complementary form under medium stringency conditions ; and
ii. Fragile X-related Epigenetic Element 2 (FREE2) comprising the nucleotide sequence set forth in SEQ ID NO:17 or a homolog thereof defined by having at least 80% nucleotide sequence identity to SEQ ID NO:17 or which hybridizes to SEQ ID NO:17 or its complementary form under medium stringency conditions,
   in the presence or absence of an agent to be tested wherein the agent is selected if it induces a change in extent of methylation.

As indicated above, in a particular embodiment, the epigenetic modification is methylation.

The methods disclosed herein allow for monitoring the treatment of a FMR genetic locus disease including FXS, FXTAS, and FXPOI in which the treatment modulates the extent of epigenetic change of the FMR genetic locus.

By "monitoring" in this context includes diagnosis of disease, monitoring progress of the disease before or after treatment, prognosis of the disease development or remission as well as the pharmacoresponsiveness or pharmacosensitivity of a subject or agent.

The assay method of the present invention may also be used alone or in combination with assays to detect extent of (CGG)ₙ expansion, such as PCR and Southern blot assays. This is particularly useful in determining homozygosity, heterozygosity and mosaicism. The assay of the present invention is also useful in population studies such as epidemiological studies as well as studies based on ethnic populations. Accordingly, the present invention further provides an *in vitro* method of identifying an epigenetic profile in a population of subjects indicative of a pathological condition associated with the FMR1, FMR4 and/or ASFMR1 genes, the method comprising screening for a change relative to a control in a statistically significant number of subjects the extent of methylation within a region of the FMR genetic locus, selected from:
i. Fragile X-related Epigenetic Element 1 (FREE1) comprising the nucleotide sequence set forth in SEQ ID NO:16 or a homolog thereof defined by having at least 80% nucleotide sequence identity to SEQ ID NO:16 or which hybridizes to SEQ ID NO:16 or its complementary form under medium stringency conditions ; and
ii. Fragile X-related Epigenetic Element 2 (FREE2) comprising the nucleotide sequence set forth in SEQ ID NO:17 or a homolog thereof defined by having at least 80% nucleotide sequence identity to SEQ ID NO:17 or which hybridizes to SEQ ID NO:17 or its complementary form under medium stringency conditions;
   wherein a change in extent of methylation is indicative of the presence of the pathological condition or a propensity to develop same in the population.

In accordance with this method the assay may comprise the further step of determining the extent of (CGG)ₙ expansion such as by PCR and/or Southern blot analysis.

The methods disclosed herein may be used to determine the status, prognosis or disease development or recovery and/or treatment options including responsiveness of the subject to pharmacological agents and/or behavioral intervention strategies.

A further embodiment of the present invention is use of primers which amplify regions of the FMR genetic locus selected from:
i. Fragile X-related Epigenetic Element 1 (FREE1) comprising the nucleotide sequence set forth in SEQ ID NO:16 or a homolog thereof defined by having at least 80% nucleotide sequence identity to SEQ ID NO:16; and
ii. Fragile X-related Epigenetic Element 2 (FREE2) comprising the nucleotide sequence set forth in SEQ ID NO:17 or a homolog thereof defined by having at least 80% nucleotide sequence identity to SEQ ID NO:17;
   in the manufacture of a diagnostic kit or device to detect methylation of the FMR locus-associated with a pathological condition associated with the FMR1, FMR4 and/or ASFMR1 genes.

A further embodiment of the present invention is directed to a kit for use in the above methods comprising primers to amplify a region within the FMR genetic locus selected from:
i. Fragile X-related Epigenetic Element 1 (FREE1) comprising the nucleotide sequence set forth in SEQ ID NO:16 or a homolog thereof defined by having at least 80% nucleotide sequence identity to SEQ ID NO:16 ; and
ii. Fragile X-related Epigenetic Element 2 (FREE2) comprising the nucleotide sequence set forth in SEQ ID NO:17 or a homolog thereof defined by having at least 80% nucleotide sequence identity to SEQ ID NO:17.

In an embodiment, the epigenetic modification relates to extent of or change in methylation at CpG and/or CpNpG sits within the selected regions of the FMR genetic locus, defined as FREE1 and FREE2.

In a particular embodiment, the primers are selected from the list consisting of SEQ ID NOs: 3 and 4 (Amplicon 1); and 11 and 12 (Amplicon 5). The nucleotide sequence of Amplicon 1 is set forth in SEQ ID NO:19; the nucleotide sequence of Amplicon 5 is set forth in SEQ ID NO:23.

Computer programs to monitor changes in epigenetic modification or profile over time that may assist in making decisions regarding treatment options including responsiveness of the subject to pharmacological agents and/or behavioral intervention strategies, also form part of the present invention.

**TABLE 1**

| ***Summary of sequence identifiers*** | |
|---|---|
| **SEQUENCE ID NO:** | **DESCRIPTION** |
| 1 | (CGG)ₙ amplification primer |
| 2 | (CGG)ₙ amplification primer |
| 3 | Forward primer for Amplicon 1 |
| 4 | Reverse primer for Amplicon 1 |
| 5 | Forward primer for Amplicon 2 |
| 6 | Reverse primer for Amplicon 2 |
| 7 | Forward primer for Amplicon 3 |
| 8 | Reverse primer for Amplicon 3 |
| 9 | Forward primer for Amplicon 4 |
| 10 | Reverse primer for Amplicon 4 |
| 11 | Forward primer for Amplicon 5 |
| 12 | Reverse primer for Amplicon 5 |
| 13 | FMR4-5' Forward primer |
| 14 | FMR4-5' Reverse primer |
| 15 | FMR4-5' probe |
| 16 | Nucleotide sequence of FREE1 |
| 17 | Nucleotide sequence of FREE2 |
| 18 | Nucleotide sequence of the FMR1 gene |
| 19 | Nucleotide sequence of Amplicon 1 |
| 20 | Nucleotide sequence of Amplicon 2 |
| 21 | Nucleotide sequence of Amplicon 3 |
| 22 | Nucleotide sequence of Amplicon 4 |
| 23 | Nucleotide sequence of Amplicon 5 |
| 24 | Nucleotide of sequence of Amplicon 1 after C/T conversion |
| 25 | Nucleotide of sequence of Amplicon 2 after C/T conversion |
| 26 | Nucleotide of sequence of Amplicon 3 after C/T conversion |
| 27 | Nucleotide of sequence of Amplicon 4 after C/T conversion |
| 28 | Nucleotide of sequence of Amplicon 5 after C/T conversion |
| 29 | Nucleotide sequence of T7 promoter inserted into primers for Amplicons 1-5 |
| 30 | Nucleotide sequence tag incorporated in 5' of primer for Amplicons 1-5 |

A list of abbreviations used herein is provided in Table 2.

**TABLE 2**

| ***Abbreviations*** | |
|---|---|
| **ABBREVIATION** | **DESCRIPTION** |
| Ab | Antibody |
| Amplicon 1 | FREE1 |
| Amplicon 5 | FREE2 |
| ASFMR1 | Antisense Fragile X mental retardation 1 gene |
| (CGG)ₙ | CGG repeat element located within 5' untranslated region of the FMRI gene |
| CpG | Cytosine and guanine separated by a phosphate (C-phosphate-G), which links the two nucleosides together in DNA |
| CpNpG | Cytosine and guanine separated by a nucleotide (N) where N is any nucleotide but guanine. The cytosine and N nucleotide are phosphorylated. |
| CVS | Cultured or uncultured Chorionic Villi Sample |
| DNA | Deoxyribonuceic acid |
| FM | Full Mutation |
| FMR | Fragile X mental retardation genetic locus comprising of FMR1 and FMR4 genes |
| FMR1 | Fragile X mental retardation 1 gene |
| FMR4 | Fragile X mental retardation 4 gene |
| FMRP | Fragile X mental retardation protein |
| FREE | Fragile X related Epigenetic Element (FREE1 and FREE2 are identified herein) |
| FREE1 | SEQ ID NO:16 |
| FREE2 | SEQ ID NO:17 |
| FXPOI | Fragile X-associated primary ovarian insufficiency |
| FXS | Fragile X Syndrome |
| FXTAS | Fragile X-associated Tremor Ataxia Syndrome |
| GZ | Gray Zone |
| HRM | Heat Resolution Melt |
| ORF | Open Reading Frame |
| PCR | Polymerase Chain Reaction |
| PM | Premutation |
| POF | Premature Ovarian Failure |

### BRIEF DESCRIPTION OF THE FIGURES

Some figures contain color representations or entities. Color photographs are available from the Patentee upon request or from an appropriate Patent Office. A fee may be imposed if obtained from a Patent Office.

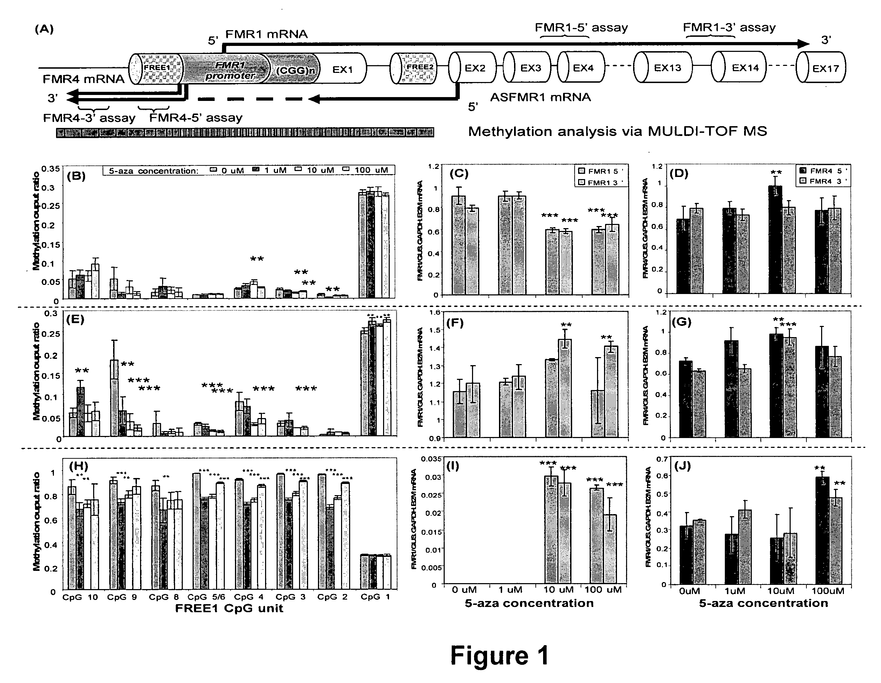

**Figure 1A** is a diagrammatic representation of the organization of the FMR genetic locus in relation to FMR1, ASFMR1 and FMR4 transcription start sites, FMR1 promoter, the Fragile X-related epigenetic elements (FREE) 1 and 2, and the locations of the real-time PCR assays target sites. FMR1 gene has 17 exons, and encodes FMRP. A CGG repeat is located within the 5' (UTR) of the FMR1 gene. The ASFMR1 and FMR4 transcripts share the bi-directional promoter with FMR1. The ASFMR1 spans the CGG expansion in the antisense direction and is also regulated by another promoter located in the exon 2 of FMR1. The FREE1 region is located upstream of the putative bi-directional promoter, and FREE2 located downstream of the CGG expansion.
**Figure 1B through J** is a graphical representation of the expression of the FMR1 and FMR4 mRNA and FREE1 methylation in lymphoblast cultures with selectively depleted DNMTI. Cell lines from (B, C, D) a male HC cell line (30 CGGs), (E, F, G) a male PM cell line (103 CGGs) and (H, I, J) a male FM cell line from an FXS individual (490 CGGs) were cultured for 3 days in standard RPMI medium/10% v/v fetal bovine serum treated with different doses of 5-aza-deoxycytidine, in triplicate wells, with half of the cells from each well taken for DNA methylation analysis, and the other half for mRNA analyses. FREE1 methylation for each sample was examined from duplicate bisulfite reactions, with single PCR products made from each conversion. The 0.1 methylation output ratio cutoff was not used for this experiment due to an increased number of technical replicates, with values expressed as means of six technical replicates per condition. Methylation and mRNA levels were assessed in the same wells using MALDI-TOF MS and real-time PCR, respectively. The error bars represent standard error (n=3 to 6). All conditions were compared to 0 µM 5-aza control: ***-p<0.001; **-p<0.05.

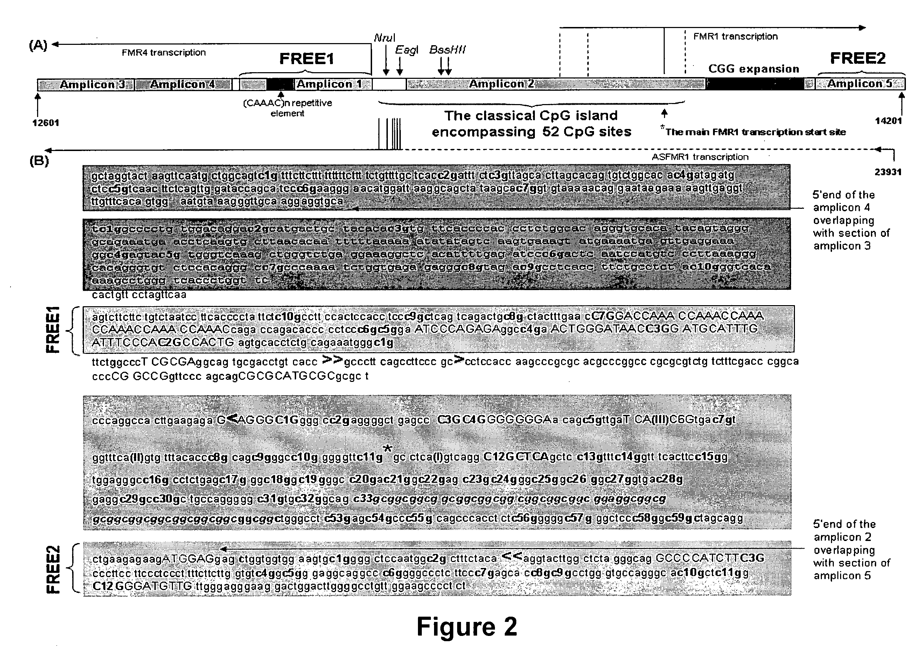

**Figure 2** is a representation of the promoter region on the FMR1 gene (sequence numbering from GenBank L29074 L38501) and the adjacent 5' and 3' loci. Primers utilized for MALDI-TOF methylation analysis targeted 5 regions at the Xq27.3 locus designated as Amplicons 1 to 5 (color coded). Individual CPG sites within each Amplicon are numbered accordingly. The 5' end of the FMR1 promoter is indicated by >> in red. The 3' end of the FMR1 promoter is indicated by << in red. The 5' end of the putative FMR1/ASFMR1 promoter is indicated by > in red, and its 3' end is indicated by <. Prominent transcription factor binding sites and methylation sensitive restriction enzyme recognition sites are indicated in capital font, and are listed/identified in Table 4. The Inr like transcription start sites are indicated by numbering in red (I to III) embedded within the sequence. The main FMR1 transcription start site is indicated by * in red. The (CGG)ₙ expansion is indicated by red italics.

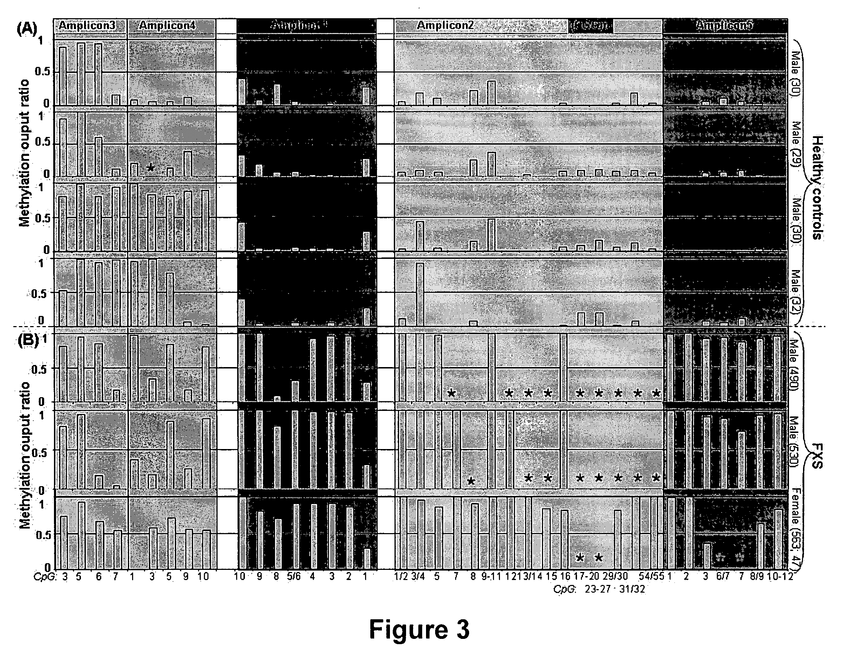

**Figure 3** is a graphical representation of the methylation pattern variation at the Xq27.3 locus between healthy controls and FXS individuals. DNA from lymphoblasts of (A) healthy controls (n=4) and (B) Fragile X syndrome effected patients (n=3). Methylation of individual CpGs, were analyzed in the 1.766 kb region 5' and 3' of the CGG expansion using 5 SEQUENOM mass spectrometry assays (see Table 3). * - represent missing values.

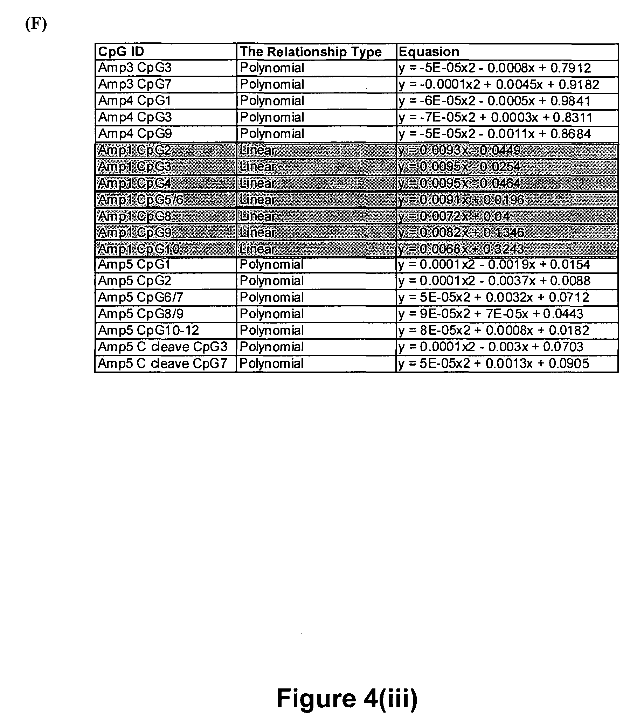

**Figures 4A through E** are graphical representations of a spiking experiment indicating the quantification limits with MALDI-TOF methylation analysis of the Fragile X (FRAX) DNA at the Xq27.3 locus (A: Amplicon 3; B: Amplicon 4; C: Amplicon 1; D: Amplicon 2; E: Amplicon 5). Healthy control DNA was spiked with FXS DNA at 1:0; 2:1; 1:1; 1:2; 0:1 ratios corresponding to 0, 33.3, 50, 66.6, 100% FXS DNA in the sample. The spiked DNA samples were analyzed using MALDI-TOF methylation analysis at five sequential regions (Amplicons) at the Xq27.3 locus (see Figure 2 for locations). The methylated vs unmethylated ratios at each analyzable CpG unit were expressed as Output Methylation Ratios on Y axis, with FXS DNA input % expressed on the X axis (each point represents mean of duplicate PCRs from a single bisulfite converted DNA mixture). **Figure 4F** is a table of equations for each CpG depicting the relationship between the methylation output (Y axis) and expected FXS DNA content (X axis).

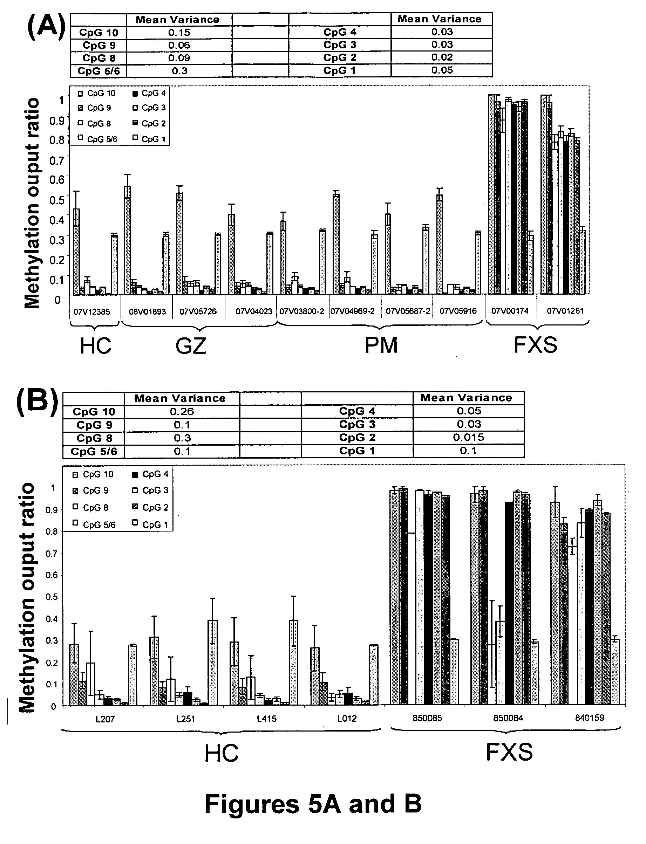

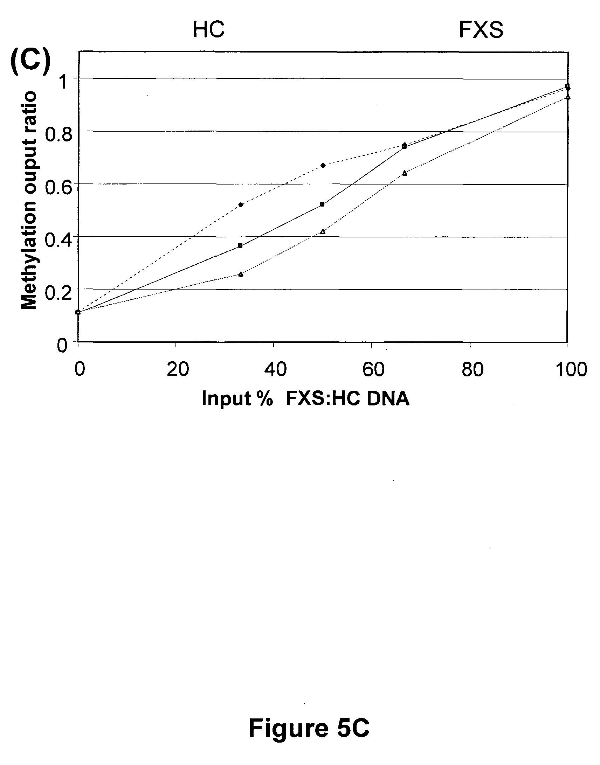

**Figures 5A through C** are graphical representations showing of inter- and intra-run technical variation and the sensitivity limits of the MALDI-TOF methylation analysis for different CpG units within the FREE1 region (Amplicon 1), obtained from multiple measurements. FREE1 methylation was examined in DNA extracted from whole blood of male CGG repeat carriers, ranging from normal size alleles to full mutation. Duplicate bisulfite reactions were made from each sample, which were amplified in duplicate PCR reactions - providing a total of 4 measurements per sample for MALDI-TOF methylation analysis. (A) The inter-run variation in the methylation output ratio for DNA from 1 healthy control (HC), 3 Gray zone (GZ), 4 premutation (PM) and 2 Fragile X Syndrome (FXS) affected individuals. CpGs 2 to 10 could be clearly used to distinguish fully methylated FXS samples from PM, GZ and HC cell lines, all largely unmethylated. However, this was not the case for CpG 1 that showed no differential methylation between the four categories. The inter-run variance in the CGG repeat carriers, ranging from normal size alleles to full mutation, was not consistent between different units within FREE1. The results for the CpG 10 were the most variable with mean variance of 0.14, while CpGs 1 to 6 were the most consistent, with mean variances between 0.02 and 0.05. CpG units 8 and 9 had intermediate levels of variability, with mean variances of 0.06 and 0.09 respectively. **(B)** The intra-run variation in the methylation output ratio for DNA from 4 healthy controls and 3 FXS individuals. CpGs 10 and 8 were also the least consistent, with the mean variances for both being ∼0.3. CpG units 1, 5/6 and 9 had intermediate levels for the intra-run variation, with mean variance of ~0.1. Similar to inter-run variation, CpG units 1 to 4 had the most consistent intra-run variation values, with mean variances between 0.02 and 0.05. One of the major factors that may have contributed to elevated inter and a run technical variation of CpG units 9 and 10, may have been presence of a silent peak for these units. Despite of this, Figure 5 clearly demonstrates that both CpGs 9 and 10 can be confidently used to distinguish FM DNA from FXS males from the smaller expansion carrier males. **(C)** is a graphical representation of the relationship between the mean methylation output across Amplicon 1 (FREE1) CpG 2 to 10 and the HC DNA spiked with 0, 33.3, 50, 66.6, 100% FXS DNA. The different lines represent 3 separate runs for the same samples, and demonstrate the intra-run variation in the spiked samples at different levels of methylation. The error bars represent the standard error.

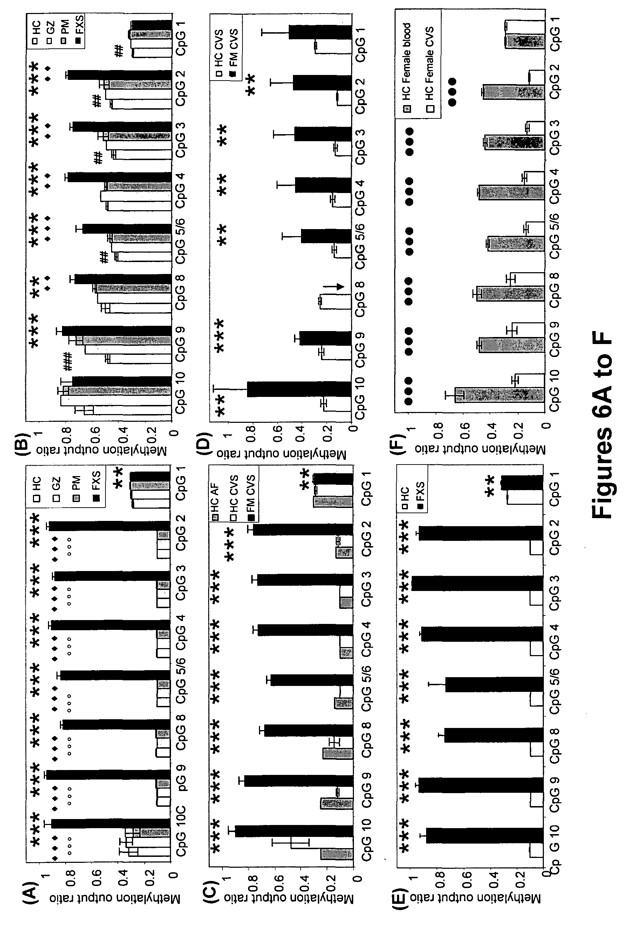

**Figure 6A through F** are graphical representations showing comparison of FREE1 methylation in different tissues between males and females. The FREE1 region methylation was examined in duplicate bisulfite reactions per sample, each amplified with a single PCR reaction. Methylation output for each sample was expressed as a mean of two technical replicates, provided that the duplicate measurements were within 35% of their mean. Based of 10% technical variance of FREE1 MALDI-TOF MS any values of 0.1 or less, were considered as 0.1. FREE1 methylation was examined in DNA extracted from the following tissues: (A) Male blood of HC (n=15), GZ (n=16), PM (n=14) and FM with FXS (n=9), (B) Female blood of HC (n=23), GZ (n=2), PM (n=8) and FM with FXS (n=5); (C) Male amniocytes of HC (n=1), CVS of HC (n=10) and FM (n=8); (D) Female CVS of HC (n=11) and FM (n=3); (E) Male EBV transformed lymphoblasts of HC (n=9) and FM with FXS (n=3); (F) Female blood of HC (n=23) and female CVS of HC (n=11). The error bars represent standard error. The arrow represents a missing value. FXS or FM carrier compared to HC ***-p<0.001; **-p<0.05; FXS or FM carrier compared to GZ ∘∘∘ - p<0.001; ∘∘-p<0.05; FXS or FM carrier compared to PM ◆◆◆ - p<0.001; ◆◆-p<0.05; PM carrier compared to HC ### - p<0.001; ##-p<0.05; HC female blood compared to HC female CVS ••• - p<0.001; •• - p<0.05. *

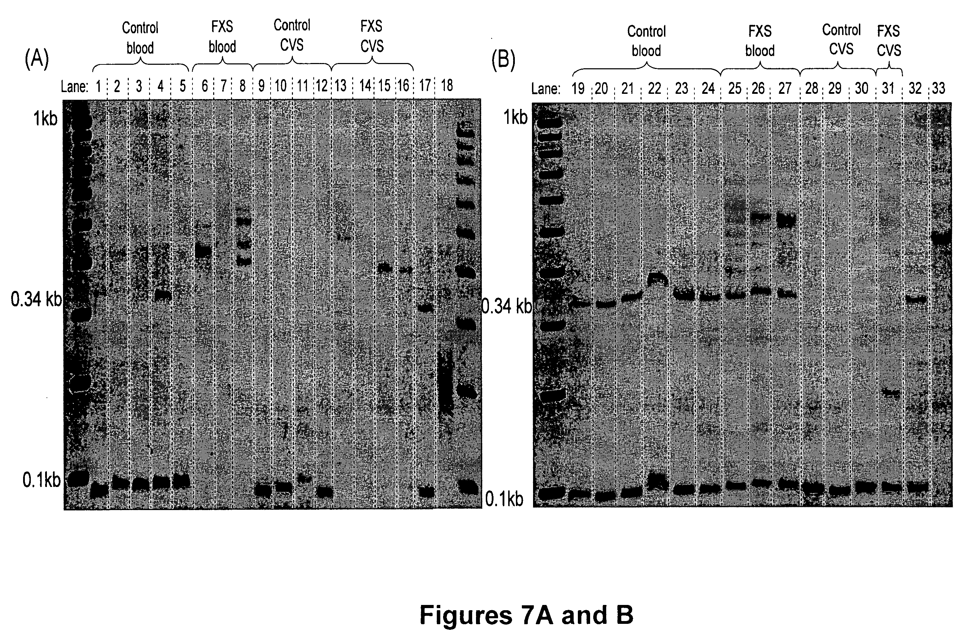

**Figures 7A through D** are graphical representations showing comparison of FREE1 and CpG island methylation using MALDI-TOF mass spectrometry and methyl sensitive Southern blot analysis in different tissues of healthy controls (HC) and Fragile X syndrome affected (FXS) individuals. (A) and (B) Southern blot fragmentation pattern resulting from NruI digestion in male (A) and female (B) DNA from HC and FXS samples. Lane 6 is a male FM male with an extra X chromosome (Klinefelter's Syndrome). Lanes 17 and 32 is DNA from a female control with 50% X-inactivation ratio. Lane 18 is a male FM male control with 0% FM allele methylation. (C) and (D) Methylation output analysis using Southern blot and MALDI-TOF in the same male (C) and female (D) samples from HC and FXS affected subjects. Methylation output ratio of 0.1 from MALDI-TOF corresponds to ≤10% methylation from Southern blot analysis.

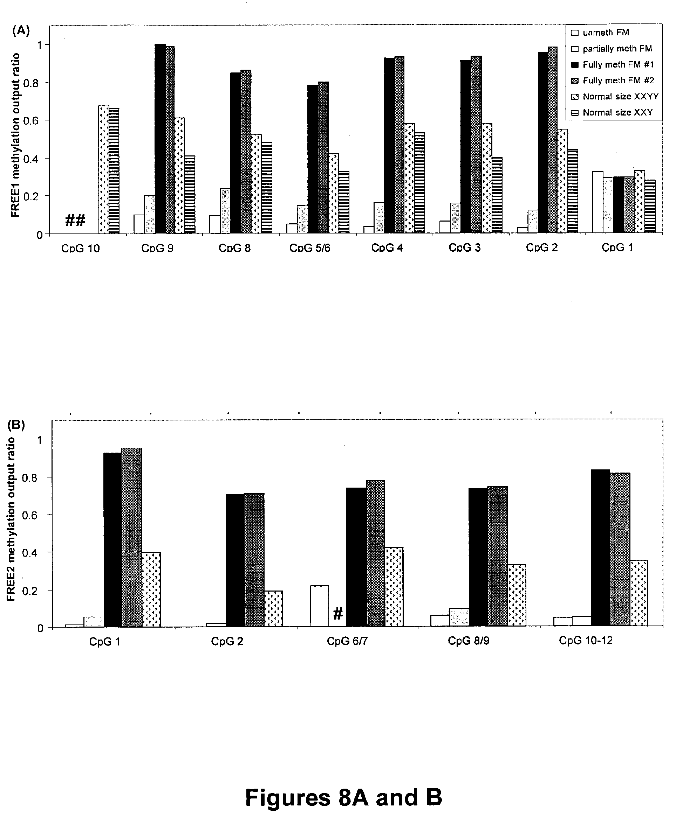

**Figures 8A and B** are graphical representations showing comparison of FREE1 and FREE2 methylation in blood of male carriers of unmethylated and partially methylated full mutation (GM) alleles, fully methylated FM alleles and Klinefelter's syndrome affected individuals with normal size CGG alleles. (A) FREE1 methylation output ratio; (B) FREE2 methylation output ratio. # represent missing values.

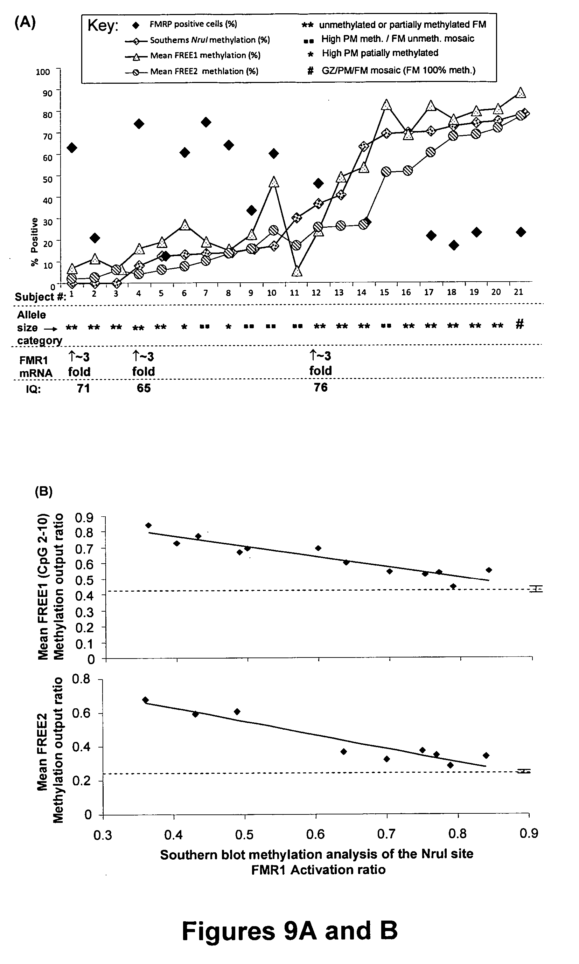

**Figures 9A and B** is a graphical representation of the relationship between FMRP positive cell counts in blood smears and Southern blot methylation analyses, FMR1 mRNA levels and standardized IQ for some of these patients was previously described by Tassone et al, Am J Med Genet, 97:195-203, 2000. (A) The mean methylation across FREE1 (CpG units 2 to 10) and FREE2 was positively correlated with Southern blot analysis and negatively correlated with the FMRP expression in 21 'high functioning' males. (B) The mean methylation across FREE1 (CpG units 2 to 10) and FREE2 was negatively correlated with the FMR1 activation ratio determined using Southern blot analysis in 12 FM and 9 FM carrier females, respectively. The broken line represents mean methylation output ratio in 11 control females analysed within the same run - baseline ± STDEV (FREE1 0.43±0.04; FREE2 0.25±0.025; Southern blot 0.39 +/- 0.045).

### DETAILED DESCRIPTION

The present invention relates to a method for identifying an epigenetic profile associated, indicative, instructive or informative of a pathological condition involving all or a portion of the FMR genetic locus as defined herein. The pathological condition may also be described as a neuropathological condition or a pathoneurological condition which encompasses neurodegenerative and neurodevelopmental disorders. Non-neurological pathologies are also contemplated herein. As described herein, the "FMR genetic locus" includes the FMR1, FMR4 and ASFMR1 genes as well as promoter and regulatory regions and introns and exons. In particular, the FMR genetic locus comprises a promoter region, a (CGG)ₙ region proximal to the promoter and exonic and intronic regions of the FMR1, FMR4 and ASFMR1 genes as depicted in Figure 1. The promoter is generally referred to as the "FMR1 promoter". The FMR locus and in particular the region upstream of the promoter (corresponding to Amplicon 1 [FREE 1]), all or part of the (CGG)ₙ region proximal to the FMRI promoter and the region downstream of the (CGG)ₙ portion of the FMR1 promoter but not including the (CGG)ₙ portion (corresponding to Amplicon 5 [FREE 2]) are subject to epigenetic change, the extent of which, is indicative or diagnostic of a pathological condition involving the FMR1, FMR4 and/or ASFMR1 genes.

Thus, disclosed herein is a method for identifying an epigenetic profile in the genome of a cell indicative of a pathological condition associated with the FMR1, FMR4 and/or ASFMR1 genes, the method comprising screening for a change relative to the control in the extent of epigenetic modification within an FMR genetic locus, the FMR genetic locus comprising an FMR1 promoter region, a (CGG)ₙ region proximal to the promoter and exonic and intronic regions of the FMR1, FMR4 and ASFMR1 genes, the epigenetic change detected in a region selected from:
i. a region upstream of the FMR1 promoter; and
ii. a region downstream of the (CGG)ₙ portion of the FMR1 promoter but not including the (CGG)ₙ portion;
wherein the extent of epigenetic indicative of the presence of the pathological condition or a propensity to develop same.

By "epigenetic profile" includes epigenetic modifications include histone modification, changes in acetylation, methylation, obiquitylation, phosphorylation, sumoylation, activation or deactivation, chromatin altered transcription factor levels and the like. Most particular, the extent of methylation is determined as well as any changes therein.

The classical FMR1 promoter is defined as beginning at 396bp 5' of the (CGG)ₙ region and ending 60bp 3' of the (CGG)ₙ region (see Figures 1 and 2). The present invention is predicated in part on a determination of the methylation or other epigenetic signature of the FMR1 promoter as well as the 5' and 3' adjacent regions with the 5' region starting 826bp away from the 5' promoter border and the 3' region encoding 190bp away from the 3' promoter border. The nucleotide sequence of the FMR1 gene is set forth in SEQ ID NO:18, the present invention extends to homologs of this gene such as genetic loci having a nucleotide sequence at least 80% identical to SEQ ID NO:18 or a nucleotide sequence capable of hybridizing to SEQ ID NO:18 under medium stringency conditions.

In a particular embodiment, five regions were analyzed at the FMR genetic locus (Xq27.3) referred to as Amplicons 1 through 5. In another embodiment, the (CGG)ₙ expansion region was analyzed.

A particularly important region in accordance with the present invention is Amplicon 1 which is a 5' epigenetic region of the FMR1 CpG island located upstream of the CGG expansion, which is termed herein "FREE1" and is defined by SEQ ID NO:16. Amplicon 5 is also another useful site which corresponds to "FREE2" which is a 3' epigenetic region of the FMR1 CpG island located downstream of the CGG expansion, and is defined by SEQ ID NO:17. The present invention extends to an isolated nucleic acid sequence of Amplicon 1 having the nucleotide sequence set forth in SEQ ID NO:16 or 19 and Amplicon 5 having the nucleotide sequence set forth in SEQ ID NO:17 or 23 or a nucleotide sequence having at least 80% identity thereto or a nucleotide sequence capable of hybridizing to these sequences or their complementary forms under medium stringency conditions. The present invention extends to portions and fragments of these regions.

Hence, the present invention provides *an in vitro* method for identifying a pathological condition associated with the FMR1, FMR4 and/or ASFMR1 genes in a mammalian subject including a human, the method comprising screening a cell from said subject for a change relative to a control in the extent of methylation within a region of the FMR genetic locus selected from:
i. Fragile X-related Epigenetic Element 1 (FREE1) comprising the nucleotide sequence set forth in SEQ ID NO:16 or a homolog thereof defined by having at least 80% nucleotide sequence identity to SEQ ID NO:16 or which hybridizes to SEQ ID NO:16 or its complementary form under medium stringency conditions ; and
ii. Fragile X-related Epigenetic Element 2 (FREE2) comprising the nucleotide sequence set forth in SEQ ID NO:17 or a homolog thereof defined by having at least 80% nucleotide sequence identity to SEQ ID NO:17 or which hybridizes to SEQ ID NO:17 or its complementary form under medium stringency conditions;
   wherein a change in extent of methylation relative to a control is indicative of the presence of the pathological condition or a propensity to develop same.

More particularly, the present invention provides *an in vitro* method for identifying in a genome of a mammalian cell including a human cell, a pathological condition associated with the FMR1, FMR4 and/or ASFMR1 genes, the method comprising extracting genomic DNA from the cell and subjecting the DNA to an amplification reaction using primers selective of a region of the FMR genetic locus selected from:
i. Fragile X-related Epigenetic Element 1 (FREE1) comprising the nucleotide sequence set forth in SEQ ID NO:16 or a homolog thereof defined by having at least 80% nucleotide sequence identity to SEQ ID NO:16 or which hybridizes to SEQ ID NO:16 or its complementary form under medium stringency conditions ; and
ii. Fragile X-related Epigenetic Element 2 (FREE2) comprising the nucleotide sequence set forth in SEQ ID NO:17 or a homolog thereof defined by having at least 80% nucleotide sequence identity to SEQ ID NO:17 or which hybridizes to SEQ ID NO:17 or its complementary form under medium stringency conditions,
and subjecting the amplified DNA to a methylation assay to determine the extent of methylation of the DNA wherein a change in extent of methylation relative to a control is indicative of the presence of the pathological condition or propensity to develop same.

In particular embodiments, the epigenetic modification is methylation of CpG and/or CpNpG sites and the assay identifies the extent of methylation change.

In accordance with the present invention, a method is provided wherein the extent of methylation provides a quantitative or semi-quantitative or qualitative indication of the length of (CGG)ₙ expansion in the FMR genetic locus and as such the expansion defines the severity of the pathological condition. The number of repeats indicate whether a subject is a healthy control or has a Gray Zone (GZ) pathology, premutation (PM) pathology or full mutation (FM) pathology. The method of the present invention may also be used in conjunction with other assays such as Southern blot or PCR to measure (CGG)ₙ expansion.

Within the meaning of the present invention a "pathological condition" or "disease condition" includes an abnormal condition including a neurodevelopmental condition or a neurodegenerative condition or a non-neurological condition as defined by objective or subjective manifestations of disease. The assay of the present invention enables a genetic determination to be made to complement other symptom-based diagnoses such as based on behavioral studies or may be made in its own right. The assay may be part of a suit of diagnostic or prognostic genetic assays of embryos, pre- and post-natal subjects. The terms "method", "assay", "system", "test", "determination", "prognostic", "diagnostic", "report" and the like may all be used to describe the methylation assay of selected regions of the FMR genetic locus. The epigenetic assay, i.e. the methylation assay, determines the epigenetic profile or extent of epigenetic change compared to a control which suggests or indicates or is instructive of a Fragile X mental retardation-like disease or condition. The present invention is also useful in population studies such as epidemiological studies including studies of ethnic populations.

Accordingly, the present invention further provides *an in vitro* method of identifying an epigenetic profile in a population of subjects indicative of a pathological condition associated with the FMR1, FMR4 and/or ASFMR1 genes, the method comprising screening for a change relative to a control in a statistically significant number of subjects the extent of methylation within a region of the FMR genetic locus, selected from the regions:
i. Fragile X-related Epigenetic Element 1 (FREE1) comprising the nucleotide sequence set forth in SEQ ID NO:16 or a homolog thereof defined by having at least 80% nucleotide sequence identity to SEQ ID NO:16 or which hybridizes to SEQ ID NO:16 or its complementary form under medium stringency conditions ; and
ii. Fragile X-related Epigenetic Element 2 (FREE2) comprising the nucleotide sequence set forth in SEQ ID NO:17 or a homolog thereof defined by having at least 80% nucleotide sequence identity to SEQ ID NO:17 or which hybridizes to SEQ ID NO:17 or its complementary form under medium stringency conditions;
   wherein a change in extent of methylation is indicative of the presence of the pathological condition or a propensity to develop same in the population. In accordance with this method the assay may comprise the further step of determining the extent of (CGG)ₙ expansion such as by PCR and/or Southern blot analysis of bisulfite converted and/or non converted DNA.

In particular, the extent of methylation or change in extent of methylation is detected and associated with the pathology condition.

A methylation map of the FMR locus has thus been constructed in accordance with the present invention using standard techniques such as high throughput mass spectrometry in the genome of various cells. Any cell type cell may be assayed. These cells include cultured or uncultured Chorionic Villi Sample (CVS) cells, lymphoblasts, blood cells, buccal cells, an amniocyte and EBV transformed lymphoblast cell lines from male and female subjects with either no symptoms or from a spectrum of Fragile X mental retardation symptoms. Two novel Fragile X-related Epigenetic Elements (FREE1 and FREE2) have been identified. FREE1 is located upstream of the putative bi-directional FMR1 promoter. FREE2 is located downstream of the putative bi-directional FMR1 promoter. It is proposed that these regions are responsible for the regulation of transcription of FMR4 and ASFMR1 and FMR1 and expression of FMRP.

The extent of methylation in CpG and/or CpNpG sites located within the region upstream of the FMR1 promoter and/or all or part of the (CGG)ₙ portion of the FMRI promoter and/or a region downstream of the (CGG)ₙ portion of the FMR1 promoter but not including the (CGG)ₙ portion closely corresponds to a healthy condition or a level of disease within the spectrum of PM to FM including GZ subjects. Furthermore, using the methylation assay, methylation levels of the FREE1 region provide fully quantitative results, which also reflect the degree of X-chromosome modification in females. This is more informative than methylation patterns of the classic promoter (Amplicon 2) only, which are highly biased due to their proximity to the CGG expansion, and can only provide qualitative assessment of methylation.

Hence, in an embodiment, the present invention contemplates a change in extent of methylation which includes an increase or decrease in extent of methylation. There may also be no change in the extent of methylation.

The present invention also demonstrates that the quantitative epigenetic pattern of the FREE1 region is significantly different in GZ and PM carriers with symptoms of a pathological condition compared to healthy controls with normal size alleles. Thus, assessment of FREE1 is proposed to be a useful biomarker for detection of the CGG expansion related neurodegenerative or neurodevelopmental disorders. This is particularly the case where the epigenetic pattern is extent of methylation.

A "normal" or "control" in the assay of the present invention may be a control genome from a healthy individual performed at the same time or the epigenetic pattern may be compared to a statistically validated standard. A healthy individual includes a subject with a (CGG)ₙ where ₙ is <40, with no clinically apparent neurological phenotype.

The present invention also explores the relationship between transcription and epigenetic profile of the FREE1 region. In an embodiment, methylated CpG sites are identified within the region that significantly correlates with decreased transcription of FREE1 in subjects with Fragile X mental retardation conditions but not normal control cell lines. FMR4/ASFMR1 transcription outside the FREE1 region was not correlated with the FREE1 region methylation, and was of a similar level in subjects with a pathological condition and normal control cell lines. In addition, partial methylation of only 2 CpG critical sites within the FREE1 correlated with increased transcription within and outside the region, as well as increased transcription of the FMR1 gene, but not translation of FMRP in the GZ/PM cell lines. Furthermore, de-methylation of the FREE1 after 5-aza-2'-deoxycytidine (5-Aza) treatment was found to increase FMR4/ASFMR1 transcription within the FREE1 in the cell lines from carriers of CGG alleles ranging from normal size to FM, indicating the potential functional role of the element's methylation in transcriptional regulation at the Xq27.3 locus.

As used herein, the terms "CpG sites" and "CpNpG sites" refer to regions up to approximately 2kb in distance upstream or downstream from the FMR1 promoter. methylation of CpG/CpNpG sites is proposed to be related to gene expression.

As used herein, the terms "subject", "patient", "individual", "target" and the like refer to any organism or cell of the organism on which an assay of the present invention is performed whether for experimental, diagnostic, prophylactic, and/or therapeutic purposes. Typical subjects include both male and female humans but the present invention extends to experimental animals such as non-human primates, (e.g., mammals, mice, rats, rabbits, pigs and guinea pigs/hamsters). The "subject" may also be referred to as a population since the present invention is useful in populations studies including epidemiological studies or assays of ethnic population. In a particular embodiment, the subject is a human. The test may be tailored to human females or human males or pre-natal humans.

The terms "Fragile X mental retardation-like condition" and FMR condition" refer to a neurological disease, disorder and/or condition characterized by one or more of the following symptoms: (1) behavioral symptoms, including but not limited to hyperactivity, stereotypy, anxiety, seizure, impaired social behavior, and/or cognitive delay; (2) defective synaptic morphology, such as an abnormal number, length, and/or width of dendritic spines; and/or (3) defective synaptic function, such as enhanced long-term depression (LTD); and/or reduced long-term potentiation (LTP); and/or impaired cognitive ability. The pathological condition is a disease, disorder, and/or condition caused by and/or associated with one or more of the following: (1) a mutation in FMR1 or FMR4 or ASFMR1; (2) defective FMR1/FMR4/ASFMR1 expression; (3) increased and/or decreased levels of FMRP; (4) defective FMRP function; (5) increased and/or decreased expression of genes or genetic functions regulated by FMR1, FMRP, FMR4 transcript or ASFMR1 transcript; (6) the increased methylation of FMR locus at CpG or CpNpG sites in the region upstream of FMR1 promoter and/or the region downstream of the (CGG)ₙ portion of the FMR1 promoter but not including the (CGG)ₙ portion; (7) an increased and/or decreased function of the FMR locus *via* miRNAs and/or members of the miRNA pathway; (8) an increased and/or decreased ability of FMRP to interact with its known target RNAs, such as RNAs encoding Racl, microtubule-associated protein IB, activity-regulated cytoskeleton-associated protein, and/or alpha-calcium/calmodulin-dependent protein kinase II; and/or (9) symptoms of FXS, FXTAS, POF, mental retardation, autism and/or autism spectrum disorders. Those of ordinary skill in the art will appreciate that the teachings of the present invention are applicable to any neurodevelopmental or neurodegenerative disorders linked, associated or otherwise influenced by the function of the FMR genetic locus or genes therein such as FMR1, FMR4 and ASFMR1. Non-neurological disorders are also contemplated herein including FXPOI.

The term "genomic DNA" includes all DNA in a cell, group of cells, or in an organelle of a cell and includes exogenous DNA such a transgenes introduced into a cell.

The present invention further enables the determination of the presence of a FM based on extent of methylation of CpG/CpNpG sites located within the FMR genetic locus. In a particular embodiment, the extent of methylation in FREE1 and FREE2 are identified.

Any methylation assay may be employed such as methylation sensitive PCR, methylation specific melting curve analysis (MS-MCA) or high resolution melting (MS-HRM) [Dahl et al, Clin Chem 53(4):790-793, 2007; Wojdacz et al, Nucleic Acids Res. 35(6):e41, 2007]; quantification of CpG methylation by MALDI-TOF MS (Tost et al, Nucleic Acids Res 31(9):e50, 2003); methylation specific MLPA (Nygren et al, Nucleic Acids Res. 33(14):e128, 2005); methylated-DNA precipitation and methylation-sensitive restriction enzymes (COMPARE-MS) [Yegnasubramanian et al, Nucleic Acids Res. 34(3):e19, 2006] or methylation sensitive oligonucleotide microarray (Gitan et al, Genome Res. 12(1):158-164, 2002), as well as *via* antibodies. Other assays include NEXT generation (GEN) and DEEP sequencing or pyrosequencing.

Insofar as the methylation assay may involve an amplification, an amplification methodology may be employed. Amplification methodologies contemplated herein include the polymerase chain reaction (PCR) such as disclosed in U.S. Patent Nos. 4,683,202 and 4,683,195; the ligase chain reaction (LCR) such as disclosed in European Patent Application No. EP-A-320 308 and gap filling LCR (GLCR) or variations thereof such as disclosed in International Patent Publication No. WO 90/01069, European Patent Application EP-A-439 182, British Patent No. GB 2,225,112A and International Patent Publication No. WO 93/00447. Other amplification techniques include Qβ replicase such as described in the literature; Stand Displacement Amplification (SDA) such as described in European Patent Application Nos. EP-A-497 272 and EP-A-500 224; Self-Sustained Sequence Replication (3SR) such as described in Fahy et al, PCR Methods Appl. 1(1):25-33, 1991) and Nucleic Acid Sequence-Based Amplification (NASBA) such as described in the literature.

A PCR amplification process is particularly useful in the practice of the present invention.

In a particular embodiment of the present invention, prior to the PCR, either essentially all cytosines in the DNA sample are selectively deaminated, but 5-methylcytosines remain essentially unchanged or essentially all 5-methylcytosines in the DNA sample are selectively deaminated, but cytosines remain essentially unchanged. Cytosine-guanine (CpG) dinucleotides and CpNpG trinucleotides are detected, allowing conclusions about the methylation state of cytosines in the CpG dinucleotides and CpNpG trinucleotide in the DNA sample. This deamination is generally performed using a bisulfite reagent. After bisulfite treatment, the 5-methylcytosines residues are converted to thymine (T), resulting in sequences SEQ ID NOs:24 to 28 (Amplicons 1 to 5, respectively) in the resulting sequence.

The sample DNA is only amplified by chosen PCR primers if a certain methylation state is present at a specific site in the sample DNA the sequence context of which is essentially complementary to one or more of the chosen PCR primers. This can be done using primers annealing selectively to bisulfite treated DNA which contains in a certain position either a TG or a CG or CNG, depending on the methylation status in the genomic DNA. Primers are designed based on particular regions around CpG and/or CpNpG sites or other FMR1 regulatory regions. Other regions include upstream of the FMR1 promoter and downstream of the (CGG)ₙ portion of the FMR1 promoter but not including the (CGG)ₙ portion.

The technology that can be alternatively employed for methylation analysis that utilizes base-specific cleavage followed by MALDI-TOF mass spectrometry on DNA after bisulfite treatment, where all the 5-methylcytosines residues are converted to thymine (T), resulting in sequences SEQ ID NOs:24 to 28 (Amplicons 1 to 5, respectively) or their alternative sequences where all unmethylated cytosines residues are not converted to thymine (T) (SEQ ID NOs: 19-23). Primers are designed based on particular regions around CpG and/or CpNpG sites or other FMR1 regulatory regions. Other regions include upstream of the FMR1 promoter and downstream of the (CGG)ₙ portion of the FMR1 promoter but not including the (CGG)ₙ portion. Particular primers are set forth in SEQ ID NOs:3 and 4 (Amplicon 1); and 11 and 12 (Amplicon 5). Before PCR amplification for each amplicon (Amplicons 1 to 5) the T7 promoter sequence (CAGTAATACGACTCACTATAGGGAGAAGGCT - SEQ ID NO:29) is incorporated on the 5'end of one of the primers and a sequence tag (AGGAAGAGAG - SEQ ID NO:30) is incorporated on the 5'end of the other primer. These primer sequences are used to amplify without bias both converted and unconverted sequences using the PCR amplification process under the medium to high stringency conditions. The PCR products are *in vitro* transcribed and subjected to base specific cleavage and fragmentation analysis using MALDI-TOF MS. The size ratio of the cleaved products provides quantitative methylation estimates for CpG sites within a target region. The shift in mass for non-methylated (NM) from methylated (M) fragments for a single CpG site is -16 daltons due to the presence of an adenosine residue in the place of a guanosine. Software is then used to calculate methylation for each fragment based on this difference in mass, where the output methylation ratios are the intensities of methylated signal/[methylated+unmethylated signal]. If the fragment size overlaps for different CpGs, their methylation output ratio is calculated based on the sum of intensities for methylated/ [methylated+unmethylated signal]. To distinguish how well the methylation output ratio for multiple fragments of a similar size represented methylation of separate CpG sites, for some amplicons both cytosine and thymidine cleave reactions can be performed (that produced fragments of different size) prior to fragment analysis. Silent peaks (S) - fragments of unknown origin, should not be taken into consideration if their size does not overlap with the fragments of interest. Methylation of CpG sites that have silent peaks (S) that overlap with the fragments of interest should be included in the analysis.

Hence, a method is provided for determining the methylation profile of one or more CpG or CpNpG sites located within the genome of a eukaryotic cell or group of cells, the method comprising obtaining a sample of genomic DNA from the cell or group of cells and subjecting the genomic DNA to primer-specific amplification within the FMR genetic locus and assaying for extent of methylation relative to a control, including a change in the extent of methylation.

A "nucleic acid" as used herein, is a covalently linked sequence of nucleotides in which the 3' position of the phosphorylated pentose of one nucleotide is joined by a phosphodiester group to the 5' position of the pentose of the next nucleotide and in which the nucleotide residues are linked in specific sequence; i.e. a linear order of nucleotides. A "polynucleotide" as used herein, is a nucleic acid containing a sequence that is greater than about 100 nucleotides in length. An "oligonucleotide" as used herein, is a short polynucleotide or a portion of a polynucleotide. An oligonucleotide typically contains a sequence of about two to about one hundred bases. The word "oligo" is sometimes used in place of the word "oligonucleotide". The term "oligo" also includes a particularly useful primer length in the practice of the present invention of up to about 10 nucleotides.

As used herein, the term "primer" refers to an oligonucleotide or polynucleotide that is capable of hybridizing to another nucleic acid of interest under particular stringency conditions. A primer may occur naturally as in a purified restriction digest or be produced synthetically, by recombinant means or by PCR amplification. The terms "probe" and "primers" may be used interchangeably, although to the extent that an oligonucleotide is used in a PCR or other amplification reaction, the term is generally "primer". The ability to hybridize is dependent in part on the degree of complementarity between the nucleotide sequence of the primer and complementary sequence on the target DNA.

The terms "complementary" or "complementarity" are used in reference to nucleic acids (i.e. a sequence of nucleotides) related by the well-known base-pairing rules that A pairs with T or U and C pairs with G. For example, the sequence 5'-A-G-T-3' is complementary to the sequence 3'-T-C-A-5' in DNA and 3'-U-C-A-5' in RNA. Complementarity can be "partial" in which only some of the nucleotide bases are matched according to the base pairing rules. On the other hand, there may be "complete" or "total" complementarity between the nucleic acid strands when all of the bases are matched according to base-pairing rules. The degree of complementarity between nucleic acid strands has significant effects on the efficiency and strength of hybridization between nucleic acid strands as known well in the art. This is of particular importance in detection methods that depend upon binding between nucleic acids, such as those of the invention. The term "substantially complementary" is used to describe any primer that can hybridize to either or both strands of the target nucleic acid sequence under conditions of low stringency as described below or, preferably, in polymerase reaction buffer heated to 95°C and then cooled to room temperature. As used herein, when the primer is referred to as partially or totally complementary to the target nucleic acid, that refers to the 3'-terminal region of the probe (i.e. within about 10 nucleotides of the 3'-terminal nucleotide position).

Reference herein to a stringency in relation to hybridization includes and encompasses from at least about 0 to at least about 15% v/v formamide and from at least about 1 M to at least about 2 M salt for hybridization, and at least about 1 M to at least about 2 M salt for washing conditions. Generally, low stringency is at from about 25-30°C to about 42°C. The temperature may be altered and higher temperatures used to replace formamide and/or to give alternative stringency conditions. Alternative stringency conditions may be applied where necessary, such as medium stringency, which includes and encompasses from at least about 16% v/v to at least about 30% v/v formamide and from at least about 0.5 M to at least about 0.9 M salt for hybridization, and at least about 0.5 M to at least about 0.9 M salt for washing conditions, or high stringency, which includes and encompasses from at least about 31% v/v to at least about 50% v/v formamide and from at least about 0.01 M to at least about 0.15 M salt for hybridization, and at least about 0.01 M to at least about 0.15 M salt for washing conditions. In general, washing is carried out Tₘ = 69.3 + 0.41 (G+C) % (Marmur and Doty, J. Mol. Biol. 5: 109, 1962). However, the Tₘ of a duplex DNA decreases by 1°C with every increase of 1% in the number of mismatch base pairs (Bonner and Laskey, Eur. J. Biochem. 46: 83, 1974). Formamide is optional in these hybridization conditions. Accordingly, particularly preferred levels of stringency are defined as follows: low stringency is 6 x SSC buffer, 0.1% w/v SDS at 25-42°C; a moderate stringency is 2 x SSC buffer, 0.1% w/v SDS at a temperature in the range 20°C to 65°C; high stringency is 0.1 x SSC buffer, 0.1% w/v SDS at a temperature of at least 65°C. Reference to at least "80% identity" includes 80, 81, 82, 83, 84, 85, 86, 87, 88, 89, 90, 91, 92, 93, 94, 95, 96, 97, 98, 99 and 100%.

The methods disclosed herein provide, therefore, a methylation or other epigenetic profile of sites within the FMR locus in a genome of a eukaryotic cell or group of cells, the methylation profile comprising the extent or level of methylation within the FMR locus. The profile may be obtained by a method comprising obtaining a sample of genomic DNA from the cell or group of cells, subjecting the digested DNA to an amplification reaction using primers selected to amplify a region of the FMR locus such as:
i. a region upstream of the FMR1 promoter; and
ii. a region downstream of the (CGG)ₙ portion of the FMR1 promoter but not including the (CGG)ₙ portion; or
   i. Amplicon 1; and
   ii. Amplicon 5; or
      i. FREE 1; and
      ii. FREE2;
         and then subjecting the amplified DNA to methylation or other epigenetic detection means to determine relative to control the extent of methylation or other epigenetic modification. A change in epigenetic modification or other epigenetic modification relative to the control is indicative of a pathological condition associated with the FMR genetic locus. Reference to "FREE1" and "FREE2" includes portions, fragments, parts, regions and domains thereof.

As indicated above, the cells may be a lymphoblast, a CVS cell, a blood cell, an amniocyte or an EBV transformed lymphoblast cell line. In addition, the methylation profile may be determined or one or both alleles of the FMR genetic locus and in selected cells where mosaicism has occurred. In particular, the extent of methylation can determine homozygosity or heterozygosity or mosaicism. Reference to "mosaicism" includes the situation wherein two or more populations of cells have different genotypes or epigenetic profiles at the FMR genetic locus.

The diagnostic assay herein can also detect heterozygosity or mosaicism where the methylation pattern is indicative of an FM in combination with an assay to detect (CGG)ₙ expansion.

The present invention also contemplates kits for determining the methylation of one or more nucleotides at one or more sites within the genome of a eukaryotic cell or group of cells. The kits may comprise many different forms but in one embodiment, the kits comprise reagents for the bisulfite methylation assay.

A further embodiment of the present invention is a kit for the use in the above methods comprising primers to amplify a region within the FMR genetic locus selected from:
i. Fragile X-related Epigenetic Element 1 (FREE1) comprising the nucleotide sequence set forth in SEQ ID NO:16 or a homolog thereof defined by having at least 80% nucleotide sequence identity to SEQ ID NO:16 ; and
ii. Fragile X-related Epigenetic Element 2 (FREE2) comprising the nucleotide sequence set forth in SEQ ID NO:17 or a homolog thereof defined by having at least 80% nucleotide sequence identity to SEQ ID NO:17.

In an embodiment, the present invention provides a use of primers which amplify regions of the FMR genetic locus selected from:
i. Fragile X-related Epigenetic Element 1 (FREE1) comprising the nucleotide sequence set forth in SEQ ID NO:16 or a homolog thereof defined by having at least 80% nucleotide sequence identity to SEQ ID NO:16; and
ii. Fragile X-related Epigenetic Element 2 (FREE2) comprising the nucleotide sequence set forth in SEQ ID NO:17 or a homolog thereof defined by having at least 80% nucleotide sequence identity to SEQ ID NO:17;
in the manufacture of a diagnostic kit or device to detect methylation of the FMR locus-associated with a pathological condition associated with the FMR1, FMR4 and/or ASFMR1 genes.

In relation to one embodiment the present invention is a kit for the use in the above methods comprising primers identified by SEQ ID NOs:3 and 4; and 11 and 12 to amplify a site within the FMR1 genetic locus, comprising a promoter region, a (CGG)ₙ region proximal to the promoter and exonic and intronic regions.

The kit may also comprise instructions for use.

Conveniently, the kits are adapted to contain compartments for two or more of the above-listed components. Furthermore, buffers, nucleotides and/or enzymes may be combined into a single compartment.

As stated above, instructions optionally present in such kits instruct the user on how to use the components of the kit to perform the various methods of the present invention. It is contemplated that these instructions include a description of the detection methods of the subject invention, including detection by gel electrophoresis.

The present invention further contemplates kits which contain a primer for a nucleic acid target of interest with the primer being complementary to a predetermined nucleic acid target. In another embodiment, the kit contains multiple primers or probes, each of which contains a different base at an interrogation position or which is designed to interrogate different target DNA sequences, In a contemplated embodiment, multiple probes are provided for a set of nucleic acid target sequences that give rise to analytical results which are distinguishable for the various probes. The multiple probes may be in microarray format for ease of use.

It is contemplated that a kit comprises a vessel containing a purified and isolated enzyme whose activity is to release one or more nucleotides from the 3' terminus of a hybridized nucleic acid probe and a vessel containing pyrophosphate. In one embodiment, these items are combined in a single vessel. It is contemplated that the enzyme is either in solution or provided as a solid (e.g. as a lyophilized powder); the same is true for the pyrophosphate. Preferably, the enzyme is provided in solution. Some contemplated kits contain labeled nucleic acid probes. Other contemplated kits further comprise vessels containing labels and vessels containing reagents for attaching the labels. Microtiter trays are particularly useful and these may comprise from two to 100,000 wells or from about six to about 10,000 wells or from about six to about 1,000 wells.

Another important application is in the high throughput screening of agents which are capable of demethylating genomes. This may be important, for example, in de-differentiating cells.

The present invention further enables a method for screening for an agent which modulates methylation of an FMR genetic locus in a mammalian cell including a human cell, said method comprising screening for a change relative to a control in the extent of methylation within a region selected from:
i. Fragile X-related Epigenetic Element 1 (FREE1) comprising the nucleotide sequence set forth in SEQ ID NO:16 or a homolog thereof defined by having at least 80% nucleotide sequence identity to SEQ ID NO:16 or which hybridizes to SEQ ID NO:16 or its complementary form under medium stringency conditions ; and
ii. Fragile X-related Epigenetic Element 2 (FREE2) comprising the nucleotide sequence set forth in SEQ ID NO:17 or a homolog thereof defined by having at least 80% nucleotide sequence identity to SEQ ID NO:17 or which hybridizes to SEQ ID NO:17 or its complementary form under medium stringency conditions;
   in the presence or absence of an agent to be tested wherein the agent is selected if it induces a change in extent of methylation.

The methods disclosed herein allow for monitoring the treatment of a FMR1 genetic locus disease including FXS, FXTAS, and FXPOI in which the treatment modulates the methylation of the FMR genetic locus, the FMR genetic locus comprising a promoter region, a (CGG)ₙ region proximal to the promoter and exonic and intronic region of the FMR1, FMR4 and ASFMR1 genes, the method comprising monitoring for a change relative to a control or a pre and post-treatment sample in the extent of methylation within the FMR genetic locus at a site disclosed herein.

By monitoring includes diagnosis, prognosis, pharmacoresponsiveness, pharmacosensitivity, level of disease progression or remission, improving or declining health of a subject and the like.

Disease conditions associated with abnormal methylation include but are not limited to FXS, FXTAS, FXPOI, autism, mental retardation, Klinefelter's syndrome, Turner's syndrome and a modified X-chromosome. Reference to a "modified" X-chromosome includes skewed X-inactivation, inversions, deletions, duplications, hybrids and any modification leading to X-chromosome inactivation.

The present invention further permits the identification of genes and promoters having CpG or CpNpG sites or other methylation-sensitive restriction sites. The identification of these sites permits identification of potential regulatory regions which can be targeted for agonists or antagonists of gene expression.

In cases where the gene is methylated and silenced in affected individuals or tissues, compounds are screened in high throughput fashion in stable cell lines or individuals to identify drugs that result in demethylation and reactivation of the affected gene. Alternatively, a normal active copy of the affected gene is transfected as a transgene into cells to correct the defect. Such transgenes are introduced with modulating sequences that protect the transgene from methylation and keep it unmethylated and transcriptionally active.

In cases where the gene is unmethylated and transcriptionally active or transcriptionally over-active in affected individuals or tissues, compounds are screened in high throughput fashion in stable cell lines to identify drugs that result in methylation and silencing of the affected gene. Alternatively, a transgene encoding a double stranded RNA homologous to the affected sequences or homologs thereof, are transfected as a transgene into cells to methylate the gene, silence it and thereby correct the defect. Such double stranded RNA-encoding transgenes are introduced with modulating sequences which protect it from methylation, keep it transcriptionally active and producing double stranded RNA.

The present invention further contemplates a computer program and hardware which monitors the changing state, if any, of extent of methylation over time or in response to therapeutic and/or behavioral modification. Such a computer program has important utility in monitoring disease progression, response to intervention and may guide modification of therapy or treatment. The computer program is also useful in understanding the association between increasing methylation and disease progression.

The computer program monitors in a quantitative or semi-quantitative manner one or more features including (a) extent of methylation or other epigenetic modification in a region of the FMR genetic locus upstream of the FMR1 promoter; (b) extent of methylation or other epigenetic modification in all or part of the (CGG)ₙ portion at the FMRI promoter; (c) extent of methylation or other epigenetic modification in CpG and/or CpNpG islands and island shores in a region downstream of (CGG)ₙ portion of the FMR1 promoter but not including the (CGG)ₙ portion; (d) length of (CGG)ₙ expansion when considered in combination with (a) and/or (b) and/or (c); (e) behavioral assessment criteria associated with normal subjects, or subjects with PM pathology, GZ pathology and FM pathology; and/or (f) extent of transcription of FMR1, FMR4 and/or ASFMR1 genes. Cognitive ability is also measured as well as the general phenotype or clinical manifestations in subjects with a neurodevelopmental or neurodegenerative condition or other conditions including FXPOI.

Thus, in accordance with the present invention, index values are assigned to the listed features which are stored in a machine-readable storage medium, which is capable of processing the data to provide an extent of disease progression or change in methylation or other epigenetic modification for a subject.

Thus, in another aspect, the invention contemplates a computer program product for operating a computer such that said computer can assess progression of a pathological condition associated with the FMR locus in a subject, the product comprising:
(1) means to assign index values to one or more of:
   (a) change in of methylation relative to a control at sites within FREE1 comprising the nucleotide sequence set forth in SEQ ID NO:16 or a homolog thereof defined by having at least 80% nucleotide sequence identity to SEQ ID NO:16 or which hybridizes to SEQ ID NO:16 or its complementary form under medium stringency conditions upstream of the FMR1 promoter;
   (b) change of methylation relative to a control at sites within FREE2 comprising the nucleotide sequence set forth in SEQ ID NO:17 or a homolog thereof defined by having at least 80% nucleotide sequence identity to SEQ ID NO:17 or which hybridizes to SEQ ID NO:17 or its complementary form under medium stringency conditions upstream of the FMR1 promoter;
   (c) length of (CGG)ₙ expansion within the FMR genetic locus when considered in combination with (a) and/or (b);
(2) means to converting index value to a code; and
(3) means to store the code in a computer readable medium.

In a related aspect, the invention extends to a computer for assessing an association between extent of methylation within the FMR locus, the FMR locus and progression of a disease condition wherein the computer comprises:
(1) a machine-readable data storage medium comprising a data storage material encoded with machine-readable data, wherein the machine-readable data comprise index values associated with the features of one or more of:
   (a) change of methylation relative to a control at sites within FREE1 comprising the nucleotide sequence set forth in SEQ ID NO:16 or a homolog thereof defined by having at least 80% nucleotide sequence identity to SEQ ID NO:16 or which hybridizes to SEQ ID NO:16 or its complementary form under medium stringency conditions upstream of the FMR1 promoter;
   (b) change of methylation or other epigenetic modification relative to a control at sites within FREE2 comprising the nucleotide sequence set forth in SEQ ID NO:17 or a homolog thereof defined by having at least 80% nucleotide sequence identity to SEQ ID NO:17 or which hybridizes to SEQ ID NO:17 or its complementary form under medium stringency conditions upstream of the FMR1 promoter;
   (c) length of (CGG)ₙ expansion within the FMR genetic locus when considered in combination with (a) and/or (b);
(2) means to converting index value to a code; and
(3) means to store the code in a computer readable medium.

In one version of these embodiments, a system including a computer comprising a central processing unit ("CPU"), a working memory which may be, e.g. RAM (random-access memory) or "core" memory, mass storage memory (such as one or more disk drives or CD-ROM drives), one or more cathode-ray tube ("CRT") display terminals, one or more keyboards, one or more input lines, and one or more output lines, all of which are interconnected by a conventional bidirectional system bus.

Input hardware, coupled to computer by input lines, may be implemented in a variety of ways. For example, machine-readable data of this invention may be inputted via the use of a modem or modems connected by a telephone line or dedicated data line. Alternatively or additionally, the input hardware may comprise CD. Alternatively, ROM drives or disk drives in conjunction with display terminal, keyboard may also be used as an input device.

Output hardware, coupled to computer by output lines, may similarly be implemented by conventional devices. By way of example, output hardware may include CRT display terminal for displaying a synthetic polynucleotide sequence or a synthetic polypeptide sequence as described herein. Output hardware might also include a printer, so that hard copy output may be produced, or a disk drive, to store system output for later use.

In operation, CPU coordinates the use of the various input and output devices coordinates data accesses from mass storage and accesses to and from working memory, and determines the sequence of data processing steps. A number of programs may be used to process the machine readable data of this invention. Exemplary programs may use, for example, the following steps:-
(1) inputting data selected from:
   (a) change in of methylation or other epigenetic modification relative to a control in FREE1 comprising the nucleotide sequence set forth in SEQ ID NO:16 or a homolog thereof or portion or part thereof defined by having at least 80% nucleotide sequence identity to SEQ ID NO:16 or which hybridizes to SEQ ID NO:16 or its complementary form under medium stringency conditions upstream of the FMR1 promoter;
   (b) change of methylation or other epigenetic modification relative to a control in FREE2 comprising the nucleotide sequence set forth in SEQ ID NO:17 or a homolog thereof or portion or part thereof defined by having at least 80% nucleotide sequence identity to SEQ ID NO:17 or which hybridizes to SEQ ID NO:17 or its complementary form under medium stringency conditions;
   (c) length of (CGG)ₙ expansion within the FMR genetic locus when considered in combination with (a) and/or (b);
   (d) general phenotype or clinical manifestations in subjects with a neurodevelopmental or neurodegenerative condition;
   (e) behavioral assessment criteria associated with normal subjects, PM subjects, GZ subjects and FM subjects;
   (f) cognitive ability;
   (g) extent of transcription of genes within the FMR locus;
(2) converting data to machine-readable codes; and
(3) outputting the codes.

Also provided is a magnetic data storage medium which can be encoded with machine readable data, or set of instructions, for monitoring change in methylation patterns or disease progression, which can be carried out by a system such as described above. Medium can be a conventional floppy diskette or hard disk, having a suitable substrate, which may be conventional, and a suitable coating, which may be conventional, on one or both sides, containing magnetic domains whose polarity or orientation can be altered magnetically. Medium may also have an opening for receiving the spindle of a disk drive or other data storage device. The magnetic domains of coating of medium are polarised or oriented so as to encode in manner which may be conventional, machine readable data such as that described herein.

Also provided is an optically readable data storage medium which also can be encoded with such a machine-readable data, or set of instructions, for designing a synthetic molecule of the invention, which can be carried out by a system. Medium can be a conventional compact disk read only memory (CD-ROM) or a rewritable medium such as a magneto-optical disk, which is optically readable and magneto-optically writable. Medium preferably has a suitable substrate, which may be conventional, and a suitable coating, which may be conventional, usually of one side of substrate.

In the case of CD-ROM, as is well known, coating is reflective and is impressed with a plurality of pits to encode the machine-readable data. The arrangement of pits is read by reflecting laser light off the surface of coating. A protective coating, which preferably is substantially transparent, is provided on top of coating.

In the case of a magneto-optical disk, as is well known, coating has no pits, but has a plurality of magnetic domains whose polarity or orientation can be changed magnetically when heated above a certain temperature, as by a laser. The orientation of the domains can be read by measuring the polarization of laser light reflected from coating. The arrangement of the domains encodes the data as described above.

The computer system of the present invention may also be linked to detection systems such as MALDI-TOF mass spectrometry machines.

Also provided is a web-based system where data on extent of methylation within the FMR locus (optionally together with clinical phenotype) are provided by a client server to a central processor which analyzes and compares to a control and optionally considers other information such as patient age, sex, weight and other medical conditions and then provides a report, such as, for example, a risk factor for disease severity or progression or status or response to treatment or an index of probability of a FMR locus-associated pathology in a subject.

Hence, knowledge-based computer software and hardware also form part of the present invention.

In particular, the assays of the present invention may be used in existing or newly developed knowledge-based architecture or platforms associated with pathology services. For example, results from the assays are transmitted *via* a communications network (e.g. the internet) to a processing system in which an algorithm is stored and used to generate a predicted posterior probability value which translates to the index of disease probability which is then forwarded to an end user in the form of a diagnostic or predictive report.

The assay may, therefore, be in the form of a kit or computer-based system which comprises the reagents necessary to detect the extent of methylation or other epigenetic modification within the FMR locus and includes computer hardware and/or software to facilitate determination and transmission of reports to a clinician.

The assay of the present invention permits integration into existing or newly developed pathology architecture or platform systems. For example, a method may be used which allows a user to determine the status of a subject with respect to an FMR locus-associated pathology, the method including:
(a) receiving data in the form of extent of methylation or other epigenetic modification at a site selected from:
   i. Fragile X-related Epigenetic Element 1 (FREE1) comprising the nucleotide sequence set forth in SEQ ID NO:16 or a homolog thereof or portion or part thereof defined by having at least 80% nucleotide sequence identity to SEQ ID NO:16 or which hybridizes to SEQ ID NO:16 or its complementary form under medium stringency conditions ; and
   ii. Fragile X-related Epigenetic Element 2 (FREE2) comprising the nucleotide sequence set forth in SEQ ID NO:17 or a homolog thereof or portion or part thereof defined by having at least 80% nucleotide sequence identity to SEQ ID NO:17 or which hybridizes to SEQ ID NO:17 or its complementary form under medium stringency conditions;
      wherein the extent of methylation or other epigenetic modification provides a correlation to the presence, state, classification or progression of the pathology; by transferring the data from the user *via* a communications network;
(c) processing the subject data *via* multivariate or univariate analysis to provide a disease index value;
(d) determining the status of the subject in accordance with the results of the disease index value in comparison with predetermined values; and
(e) transferring an indication of the status of the subject to the user *via* the communications network. Reference to the multivariate or univariate analysis includes an algorithm which performs the multivariate or univariate analysis function.

Conveniently, the method generally further includes:
(a) having the user determine the data using a remote end station; and
(b) transferring the data from the end station to the base station *via* the communications network.

The base station can include first and second processing systems, in which case the method can include:
(a) transferring the data to the first processing system;
(b) transferring the data to the second processing system; and
(c) causing the first processing system to perform the multivariate analysis function to generate the disease index value.

The method may also include:
(a) transferring the results of the multivariate or univariate analysis function to the first processing system; and
(b) causing the first processing system to determine the status of the subject.

In this case, the method also includes at least one of:
(a) transferring the data between the communications network and the first processing system through a first firewall; and
(b) transferring the data between the first and the second processing systems through a second firewall.

The second processing system may be coupled to a database adapted to store predetermined data and/or the multivariate analysis and/or univariate analysis function, the method including:
(a) querying the database to obtain at least selected predetermined data or access to the multivariate or univariate analysis function from the database; and
(b) comparing the selected predetermined data to the subject data or generating a predicted probability index.

The second processing system can be coupled to a database, the method including storing the data in the database.

The method can also include having the user determine the data using a secure array, the secure array of elements capable of determining the extent of methylation within the FMR locus and having a number of features each located at respective position(s) on the respective code. In this case, the method typically includes causing the base station to:
(a) determine the code from the data;
(b) determine a layout indicating the position of each feature on the array; and
(c) determine the parameter values in accordance with the determined layout, and the data.

The method can also include causing the base station to:
(a) determine payment information, the payment information representing the provision of payment by the user; and
(b) perform the comparison in response to the determination of the payment information.

Also provided is a base station for determining the status of a subject with respect to a pathology associated with the FMR locus, the base station including:
(a) a store method;
(b) a processing system, the processing system being adapted to;
(c) receive subject data from the user *via* a communications network, the data; including extent of methylation within the FMR locus wherein the level or methylation or epigenetic modification relative to a control provides a correlation to the presence, state, classification or progression of the pathology;
(d) performing an algorithmic function including comparing the data to predetermined data;
(e) determining the status of the subject in accordance with the results of the algorithmic function including the comparison; and
(f) output an indication of the status of the subject to the user *via* the communications network.

The processing system can be adapted to receive data from a remote end station adapted to determine the data.

The processing system may include:
(a) a first processing system adapted to:
   (i) receive the data; and
   (ii) determine the status of the subject in accordance with the results of the multivariate or univariate analysis function including comparing the data; and
(b) a second processing system adapted to:
   (i) receive the data from the processing system;
   (ii) perform the multivariate or univariate analysis function including the comparison; and
   (iii) transfer the results to the first processing system.

The determination of the extent of methylation or other epigenetic modification within the FMR locus at a site selected from:
i. Fragile X-related Epigenetic Element 1 (FREE1) comprising the nucleotide sequence set forth in SEQ ID NO:16 or a homolog thereof or portion or part thereof defined by having at least 80% nucleotide sequence identity to SEQ ID NO:16 or which hybridizes to SEQ ID NO:16 or its complementary form under medium stringency conditions ; and
ii. Fragile X-related Epigenetic Element 2 (FREE2) comprising the nucleotide sequence set forth in SEQ ID NO:17 or a homolog thereof or portion or part thereof defined by having at least 80% nucleotide sequence identity to SEQ ID NO:17 or which hybridizes to SEQ ID NO:17 or its complementary form under medium stringency conditions;
enables establishment of a diagnostic or prognostic rule based on the extent of methylation relative to controls. Alternatively, the diagnostic or prognostic rule is based on the application of a statistical and machine learning algorithm. Such an algorithm uses relationships between methylation profiles and disease status observed in training data (with known disease status) to infer relationships which are then used to predict the status of patients with unknown status. An algorithm is employed which provides an index of probability that a patient has an FMR locus-associated pathology. The algorithm performs a multivariate or univariate analysis function.

Hence, a diagnostic rule based on the application of statistical and machine learning algorithms may be developed. Such an algorithm uses the relationships between epigenetic profile and disease status observed in training data (with known disease status) to infer relationships which are then used to predict the status of patients with unknown status. Practitioners skilled in the art of data analysis recognize that many different forms of inferring relationships in the training data may be used without materially changing the present invention.

A knowledge base of training data comprising extent of methylation within the FMR genetic locus from a subject with an FMR-associated pathology may be used to generate an algorithm which, upon input of a second knowledge base of data comprising levels of the same biomarkers from a patient with an unknown pathology, provides an index of probability that predicts the nature of unknown pathology or response to treatment.

The term "training data" includes knowledge of the extent of methylation relative to a control. A "control" includes a comparison to levels in a healthy subject devoid of a pathology or is cured of the condition or may be a statistically determined level based on trials.

Methylation, including the epigenetic profile of the FMR genetic locus or sites therein may be used to assess or determine the status of a subject with respect to disease, to stratify a subject relative to normal controls or unhealthy subjects, to provide a prognosis of recovery or deterioration and/or to determine the pharmacoresponsiveness or pharmacosensitivity of a subject to treatment or an agent for use in treatment and/or determine applicability for other treatment options including behavioural intervention, and the like.

Hence, also provided herein is a method of allowing a user to determine the status, prognosis and/or treatment response of a subject with respect to an FMR locus-associated pathology, the method including:
(a) receiving data in the form of extent of methylation or other epigenetic modification at a site selected from:
   i. Fragile X-related Epigenetic Element 1 (FREE1) comprising the nucleotide sequence set forth in SEQ ID NO:16 or a homolog thereof or portion or part thereof defined by having at least 80% nucleotide sequence identity to SEQ ID NO:16 or which hybridizes to SEQ ID NO:16 or its complementary form under medium stringency conditions ; and
   ii. Fragile X-related Epigenetic Element 2 (FREE2) comprising the nucleotide sequence set forth in SEQ ID NO:17 or a homolog thereof or portion or part thereof defined by having at least 80% nucleotide sequence identity to SEQ ID NO:17 or which hybridizes to SEQ ID NO:17 or its complementary form under medium stringency conditions,
      wherein the extent of methylation or epigenetic modification provides a correlation to the presence, state, classification or progression of the pathology; by transferring the data from the user *via* a communications network;
(c) processing the subject data *via* multivariate or univariate analysis to provide a disease index value;
(d) determining the status of the subject in accordance with the results of the disease index value in comparison with predetermined values; and
(e) transferring an indication of the status of the subject to the user *via* the communications network.

The present invention is further described by the following non-limiting Examples. In these Examples, materials and methods as outlined below were employed.

### Patient Samples

FXS and premutation carrier EBV transformed lymphoblast cell lines were obtained from the tissue culture storage repository of the Murdoch Childrens Research Institute, Melbourne, Victoria, Australia or purchased from Coriell.

All diagnostic whole blood, amniocyte samples and CVS, taken for routine Fragile X testing for VCGS Pathology and used in this study, were de-identified upon arrival. Bloods were taken from males and females as part of FXS cascade testing. CVS (11 to 15 weeks) was taken trans- abdominally from women 11-15 weeks gestation using ultrasound guidance. An amount of 12-15mg of each CVS was suspended in 1ml Dulbecco's phosphate buffered saline (PBS)/Ca²⁺Mg²⁺ and transferred into 25cm² flasks containing 5ml PBS, 10ml penicillin (10,000IU/ml)/Streptomycin (10,000IU/ml), 10 ml heparin (1,000IU/ml), and stored at 4 °C until DNA extraction. To achieve mesenchymal cell line enrichment a portion of the CVS samples was cultured for 7 days in Amniomax II medium, as per manufacturer's instructions (Invitrogen), until DNA was extracted. CVS cells were only used if taken from back-up cultures after a successful katyotype analysis.

### Tissue culture and 5 aza treatment

Lymphoblast cell lines were established by Epstein-Barr virus (EBV) transformation from peripheral blood of individuals with: 30, 170 and 490 repeats. The cell lines were treated with 5 aza based on a previously described protocol (Pietrobono *et al,* 2002, *supra*). Briefly, a 10mM stock solution of 5-aza (Sigma-Aldrich) was initially prepared in sterile water. The cell-viability counts and cell composition were determined by trypan blue exclusion, using a hemocytometer. Cells were then resuspended at a density of 106 cell/ml in RPMI medium (Sigma St Luis, MO, USA)/10% v/v Fetal bovine serum (CSL) at 37°C, 5% v/v CO₂, supplemented with of 5-aza stock and thoroughly resuspended. The cells were cultured in triplicate wells for each of the four final 5-Aza concentrations of: 0, 1, 10 and 100µM. After 3 days, cells from half the volume of each well were washed in PBS and resuspended in RNA lysis buffer, (10 µl β-mercaptoethanol /1 ml of RLT buffer from Qiagen, Hilden, Germany), and frozen -800C until RNA extraction, the other half was washed and frozen at -200C until DNA extraction.

### DNA extraction

DNA for CGG repeat size PCR and methylation analysis was obtained either from 200µl venous blood samples anti-coagulated with EDTA or from EBV transformed lymphoblasts 1 to 5x10⁶ cells per sample and extracted using a BIO ROBOT M48 DNA Extractor, as per manufacturer's instructions (Qiagen Inc., Hilden, Germany). DNA for Southern blot or methylation analysis was extracted from 3ml blood samples anti-coagulated with EDTA or from EBV transformed lymphoblasts 5 to 10x10⁶ cells per sample or CVS from either freshly harvested or cultured samples.

### CGG repeat size PCR amplification

CGG repeat size for all samples was initially assessed using a fully validated PCR assay with precision of +/- one triplet repeat across the normal and GZ ranges, performed using a fragment analyzer (MegaBace, GE Healthcare), with the higher detection limit of 170 repeats, as previously described *(*Khaniani et al, Mol Cytogenet 1(1):5, 2008). Briefly, PCR amplifications were performed using primers:
- c (5'- GCTCAGCTCCGTTTCGGTTTCACTTCCGGT-3 [SEQ ID NO:1]); and
- f (5'AGCCCCGCACTTCCACCACCAGCTCCTCCA-3' [SEQ ID NO:2]),

(Fu et al, Cell 67(6):1047-1058, 1991) in a total volume of 25 µl containing 50ng of genomic DNA, 0.75 pmol of each primer, 8 µl of 5xQ-Solution (Qiagen Inc., Hilden, Germany), 2.5 µl of 10×PCR Buffer and 1 unit of HotStarTaq Plus DNA polymerase (Qiagen Inc., Hilden, Germany) in a Gene Amp@ PCR System 9700. The PCR cycling profile was as follows: initial denaturation at 98°C for 5 minutes; 35 cycles at 98°C for 45 seconds, 70°C for 45 seconds, and 72°C for 2 minutes, and a final extension at 72°C for 10 minutes. Alleles were sized by capillary electrophoresis using an automatic sequencer (MegaBACETM 1000 - GE HealthCare Amersham) with size standards (HealthCare) and controls of lengths 10, 23, 29, 30, 52 and 74 repeats determined by sequencing in-house or obtained from Coriel Cell Repositories web site (http://www.phppo.cdc.gov/dls/genetics/qcmaterials/).

### CGG repeat size by Southern Blot

CGG sizes were assessed using a fully validated Southern Blot procedure with appropriate normal and abnormal controls for samples where the products could not be amplified using PCR (Fu *et al* 1991, *supra*; Francis et al, Mol Diagn 5(3):221-225, 2000). Briefly, 5mg of DNA was digested with Pst1 (Boehringer Mannheim, Castle Hill, Australia), separated on 1% w/v agarose gels, and analyzed by Southern blot hybridization. The FMR-1 gene was detected using Southern blot analysis with probe Fxa3 and an X chromosome control probe, pS8 (Yu et al, Science 252(5010):1179-1181, 1991). Probes were labeled using random oligonucleotide priming (Boehringer, Mannheim) with [a32-P]CTP (NEN Dupont, Boston, MA). Autoradiography was performed at -80°C, with intensifying screens and Kodak XAR films (Sigma-Aldrich).

### Methylation sensitive Southern Blot analysis

Methylation of the classical FMR1 CpG island was assessed using a fully validated methyl sensitive Southern Blot procedure with appropriate normal and abnormal controls, as previously described (Tassone et al, J. Mol. Diagn 10:43-49, 2008). Briefly, EcoRI and NruI digestion was performed on 7 to 9µg of DNA, and separated on a 0.8% w/v agarose/Tris acetate EDTA (TAE) gel. The DNA was denatured with HCL and NAOH, transferred to a charged nylon membrane and analysed by Southern blot hybridization. The FMR1 alleles were detected using Southern blot analysis with probe StB12.3, labeled with Dig-11-dUTP by PCR (PCR Dig Synthesis kit; Roche Diagnostics). Autoradiography was performed with intensifying screens and Fuji Medical X-Ray film (Bedford, UK) and FMR1 methylation values for the expanded alleles were calculated as preciously described (Tassone *et al*, 2008 *supra*). The FMR1 activation ratios for female samples were calculated based on the following formula: optically scanned density of the 2.8kb band / combined densities of the 2.8kb and 5.2kb bands (where the 2.8kb band represents the proportion of normal active X and the 5.2kb band represents the proportion of normal inactive X), as preciously described (de Vries et al, Am JHum Genet. 58:1025-1032, 1996).

### MALDI-TOF methylation analysis

### Bisulfite Treatment

Bisulfite treatment of genomic DNA at 0.5µg per sample was performed using XCEED kit from MethylEasy (Human Genetic Signatures, Sydney, Australia) for sample sets of n<40. For sample sets n>40 96 well Methylamp kit from Epigentek (Brookly, NY, USA) was used. Duplicate bisulfite reactions were made from each sample, and six of the same control DNA samples spiked with DNA from an FXS patient cell line at 0, 33.3, 50, 66.6 or 100% were included as standards within each run, as an indicator of the inter-run variation in the degree of bisulfite related bias. Protocols were performed according to the manufacturer's instructions. Briefly, for the MethylEasy conversion, 20µl of genomic DNA (0.5µg total) was mixed with 2.2µl of 3µl NaOH, and incubated at 37°C for 15 minutes, then denatured by 45 minute incubation at 80 °C. 240µl of the reagent #3 (XCEED kit, Human Genetic Signatures, Sydney, Australia) were then added to the mixture, which was transferred into the purification column and spun down at 10,000g for 1 minute. The captured DNA was then washed in Reagent #4 (XCEED kit, Human Genetic Signatures, Sydney, Australia), and DNA eluted twice by placing 50µl of the pre-warmed solution #5 (XCEED kit, Human Genetic Signatures, Sydney, Australia) onto column membrane, which was incubated for 1 minute at room temperature, and spun down at 10,000g for 1 minute. The eluted DNA was then incubated at 95°C for 20 minutes, with resulting final concentration at ~20ng/µl per sample.

For the Methylamp conversion, 7µl of genomic DNA (0.5µg total) was mixed with 5µl of the CF3 (Methylamp kit, Epigentek, Brookly, NY, USA) solution diluted 1:10 in distilled water, in each well of the 96 well plate. The DNA was denatured by placing the plate at 65°C for 90 minutes. It was then captured in the filter plate and washed in 150µl of the CF5 solution (Methylamp kit, Epigentek, Brookly, NY, USA), then twice in 250µl of 80% v/v ethanol. The filter plate was then incubated in the CF3/90% ethanol solution, and washed twice in 90% v/v ethanol, as per manufacturer's instructions. The modified and cleaned DNA was then eluted with 40µl of the CF6 solution (Methylamp kit, Epigentek, Brookly, NY, USA), with resulting converted DNA final concentration at ~20ng/µl per sample. For the short term storage the converted DNA was kept at -20°C, and for storage of more than 3 months it was kept at -80°C.

### PCR and in vitro transcription

The primers used to amplify the target regions and the annealing temperatures are listed in Tables 3 and 4. Each bisulfite converted sample was analyzed in duplicate PCR reactions, carried out in a total volume of 5µl using 1pmol of each primer, 40µM dNTP, 0.2 U Hot Star Taq DNA polymerase (Qiagen Inc., Hilden, Germany), 1.5mM MgCl₂ and buffer supplied with the enzyme (final concentration IX). The reaction mix was pre-activated for 15min at 95°C, followed by 45 cycles of amplification at 94°C for 20s, primer specific annealing (Tables 3 and 4) for 30s and 72°C for 1 min followed by 72°C for 3 min. The PCR products were run on 1.5% w/v agaose gel to confirm successful PCR amplification and efficiency. The DNA was then cleaned up and the T or C-cleavage reactions were carried out (T-cleave for Amplicons 1 to 5, C-cleave for Amplicon 5 only) as per manufacturer's instructions (SEQUENOM, San Diego, CA). Briefly, unincorporated dNTPs were dephosphorylated by adding 1.7µl H₂O and 0.3U Shrimp Alkaline Phosphatase (SAP) [SEQUENOM, San Diego] to PCR products, which were incubated at 37°C for 20min, and 10min at 85°C to heat-inactivate the SAP. The transcription was performed on 2µl of template DNA in the 6.5ul reaction consisting of 20 U of the T7 R&DNA polymerase (Epicentre, Madison, WI) to incorporate either dCTP or dTTP; Ribonucleotides at 1nM and the dNTP substrate at 2.5mM, with other components used as recommended (SEQUENOM, San Diego). RNase A (SEQUENOM, San Diego) was then added to the mix to cleave the *in vitro* transcript. The mix was diluted to 27µl in H₂O, and 6mg CLEAN Resin (SEQUENOM, San Diego, CA) was added for conditioning of the phosphate backbone prior to MALDI-TOF MS. The SEQUENOM Nanodispenser was then used to spot the samples onto a SpectroCHIP for subsequent analysis. MassARRAY mass spectrometer (Bruker-SEQUENOM) was then used to collect mass spectra, which were analysed using the EpiTYPER software (Bruker-SEQUENOM). The calculation of the output methylation ratios for each CpG unit were based on the ratio of the signal intensities for the fragment from a methylated CpG unit/[methylated+unmethylated CpG units]. Further details are described in (Godler et al, Hum Mol Genet, 2010, [Epub ahead of print] doi: 10.1093/hmg/ddq1037).

**TABLE 3**

| ***Amplicon details used for MALDI*-*TOF methylation analysis of the regions proximal to the CGG expansion at the Xq27.3 locus*** | | | | | |
|---|---|---|---|---|---|
| **Amplicon No.** | **Annealing Temperature** | **Size (bp)** | **Distance(bp) from:** | **Primer sequence** | **SEQ ID NO:** |
| 1 | 10 cycles of: 55-52 °C touchdown PCR; 35 cycles of: 94°C for 20s, 54°C for 30s, 72°C for 1min, 72°C for 3min | 220 | 602 - | Fw: 5'- TGTTTATTTTTGTAGAGGTGTATTTAGTGG -3' | 3 |
| | | | 5'(CGG)ₙ | Rv: 5'- CTTCTATCTAATCCTTCACCCCTATTCT -3' | 4 |
| 2 | 10 cycles of: 55-52 °C touchdown PCR; 35 cycles of: 94°C for 20s, 54°C for 30s, 72°C for 1min, 72°C for 3 min | 312+(CGG)ₙ | 250 -5'(CGG)ₙ | Fw: 5'- TTTAGGTTATTTGAAGAGAGAGGG -3' | 5 |
| | | | | Rv: 5'- CTCCATCTTCTCTTCAACCCTACTA -3' | 6 |
| 3 | 10 cycles of: 56-52 °C touchdown PCR; 35 cycles of: 94°C for 20s, 53°C for 30s, 72°C for 1min, 72°C for 3min | 247 | 1226 -5'(CGG)ₙ | Fw: 5'- TTTTGTTAGGTATTAAGTTTAATGTTGGT-3 | 7 |
| | | | | Rv: 5'- CCTTACAACCCTTTACATTCCACTATA -3' | 8 |
| 4 | 10 cycles of: 56-52 °C touchdown PCR; 35 cycles of: 94°C for 20s, 53°C for 30s, 72°C for 1min, 72°C for 3min | 368 | 965 -5'(CGG)ₙ | Fw: 5'- AATGTAAAGGGTTGTAAGGAGGTGT -3' | 9 |
| | | | | Rv: 5'- CAACCAAAATAACCCAAACTTTTATAACC -3' | 10 |
| 5 | 45 cycles of: 94°C for 20s, 55.8°C for 30s, 72°C for 1min, 72°C for 3min | 240 | 62 -3'(CGG)ₙ | Fw: 5'- TTGAAGAGAAGATGGAGGAGTTGG -3' | 11 |
| | | | | Rv: 5'- AAAAAAACTTCCAACAAACCCC -3' | 12 |

### RNA extractions and quality assessments

T otal RNA was extracted and purified using the Rneasy extraction kit, as per manufacturer's instructions (Qiagen Inc., Hilden, Germany). RNA concentrations were measured in triplicate using a NanoDrop ND-1000 Spectrophotometer, with purity being determined by the A260/A280 ratio using the expected values between 1.8 and 2. Total RNA quality and the degree of DNA contamination was also assessed using capillary electrophoresis Standard Sens Kit (Bio-rad), which involved descriptive comparison of chromatographic features based on previous publications using this system (Fleige and Pfaffl, Mol Aspects Med 27(2-3):126-139, 2006). Each RNA sample was then diluted to 30ng/ul, to be used in for reverse transcription real-time PCR analysis, where mRNA quality at the Xq27.3 region was initially assessed by examining the relationship between 5' and 3' levels of FMR1 mRNA.

### Standard reverse transcription real-time PCR

Reverse transcription was performed one reaction per sample using the Multiscribe Reverse Transcription System, 50 units/µl (Applied Biosystems). The 7900HT Fast Real Time PCR (Applied Biosystem's) was used to quantify FMR1-5', FMR1-3', FMR4-5', FMR4-3', GAPDH, B2M, and GUS, using the relative standard curve method. The target gene and the internal control gene dynamic linear ranges were performed on a series of doubling dilutions of an RNA standard (160-4 ng/µl). Since, both FMR4-5' and FMR4-3' assays do not target an exon/exon boundary, to minimize the impact of potential DNA contamination on the expression results, a no reverse transcription enzyme control was included for every sample. The difference between the plus and minus no reverse transcriptase control was considered as the FMR4 expression value for each sample. Previously published sequences were be used for primers and probe for: FMR1-5' and GUS (32); FMR1-3' (41); FMR4-3'. The FMR4-5' Forward (CCGCGGAATCCCAGAGA) [SEQ ID NO:13] and Reverse (CAGTGGCGTGGGAAATCAA) [SEQ ID NO:14] primers and probe (TGGGATAACCGGATGCA) [SEQ ID NO:15] sequences were designed using Primer Express 3.0 (Applied Biosystems). FMR1-5', FMR1-3', FMR4-5', FMR4-3' primers and probes were be used at concentrations of 18µM and 2µM, respectively. GAPDH and B₂M primer/probe mixes will be obtained from PrimerDesign (PerfectProbe ge-PP-12-hu kit) and used at concentration of 2µM. All of the above assays were single-plexed, with each sample assayed in duplicate 10 µl PCR reactions. The reactions consisted of 5.8 mM MgCl2, 1 µl Buffer A (Applied Biosystems), 3.35 µl Rnase-free water, 1.2 mM dNTPs, 0.01 units/µl of AmpliTaq Gold, 0.5µl of TaqMan probe and 0.5µl forward and 0.5µl reverse primers, and 1µl of the reverse transcription (cDNA) reaction. The annealing temperature for thermal cycling protocol was 60°C for 40 cycles. The samples were quantified in arbitrary units (au) in relation to the standard curves performed on each plate, standardized to the mean of the 3 internal control genes (GUS, GAPDH and B₂M).

### Western blot FMRP analysis

FMRP quantification was performed on 20µg of total protein lysate, using appropriate FXS negative controls and standard curve samples, using standard Western blot protocol. Briefly, mouse FMR1 monoclonal antibody raised against a partial recombinant FMRP protein and goat anti-mouse HRP conjugated antibody (Jackson Immuno Research) were used as primary and secondary antibodies respectively. A mouse monoclonal [DM1A] anti-actin (Santa Cruz Biotechnology) antibody was be used as a loading control. An ECL system (GE Healthcare) will be used to develop the blot, and the results were be expressed as mean band density ratios between total FMRP and actin, determined using ImageQuant software (Molecular Dynamics).

### EXAMPLE 1

### Mapping methylation of the FMR genetic locus using high throughput mass spectrometry

The structure of the FMR genetic locus is shown in Figure 1A and comprises the FMR1 promoter, and FMR1, FMR4 and ASFMR1 genes.

The FMR promoter is defined as beginning at 396bp 5' of the (CGG)ₙ region, and ending 60bp 3' of the (CGG)ₙ region (Figures 1A and 2). The promoter encompasses a52 CpG site island (Piertrobono et al, Nucleic Acids Res 30:3278-3285, 2009) and includes a large number of regulatory motifs (Table 4). In accordance with the present invention, the methylation patterns of this promoter were mapped, as well as the 5' and 3' adjacent regions - with the 5' region starting 826bp away from the 5' promoter border, and the 3' region ending 190bps away from the 3' promoter border. Five targeted regions, utilized for MALDI-TOF methylation analysis of the Xq27.3 locus, were referred in Figure 2 as Amplicons 1 to 5.

Regions identified as biologically significant showed consistent differences in methylation between healthy controls and FXS samples. The main FMR1 transcription start site and the alternative Inr like start site I, was found between CpGs 11 and 12, Inr like start site II was between CpGs 7 and 8, and the Inr like start site III was located between CpGs 5 and 7 of the Amplicon 2 (Figure 2 and 3). One of the two main FMR1 regulatory transcription factor binding sits previously reported to be functionally influenced by methylation-USF1/2 binding site Footprint I (Kumari and Usdin, J. Biol. Chem. 276:4357-4364, 2001) was found at the CpG unit 6 of the Amplicon 2. the analysis of HC and FXS DNA demonstrated that these CpG units (5 to 12) surrounding the four FMR1 transcription start sites and the USF1/2 binding site Footprint I were fully methylated in FXS, but not in healthy control DNA. There was also a weak methylation signal detected for CpGs 8-11 of Amplicon 2 in the HC cell lines.

From the analysis of the proximal Amplicons, two novel regions adjacent to the classical FMR1 CpG island were identified. Amplicons 1 (Fragile X related Epigenetic Elements 1, FREE1) and 5 (Fragile X related Epigenetic Elements 2, FREE2) were consistently hypermethylated in FXS samples but unmethylated or partially methylated in HC cell lines. These regions have not been previously shown to have any biological significance in FXS or any other FRM1/CGG expansion related disorders. There are also no previously described functional regulatory motifs for these regions. Thus, the sequences for putative transcription factor binding sites with TFSEARCH (Heinemeyer et al, Nucleic Acids Res 26:362-367, 1998) were examined using vertebrate matrices and a standard threshold of 0.85. Subsequently, a number of shared prominent putative transcription factor binding sites were identified at both locations (Table 4).

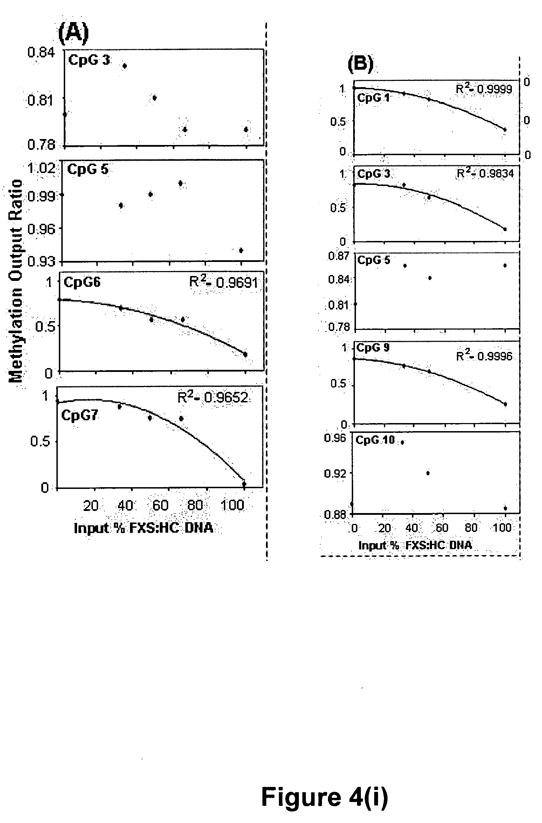

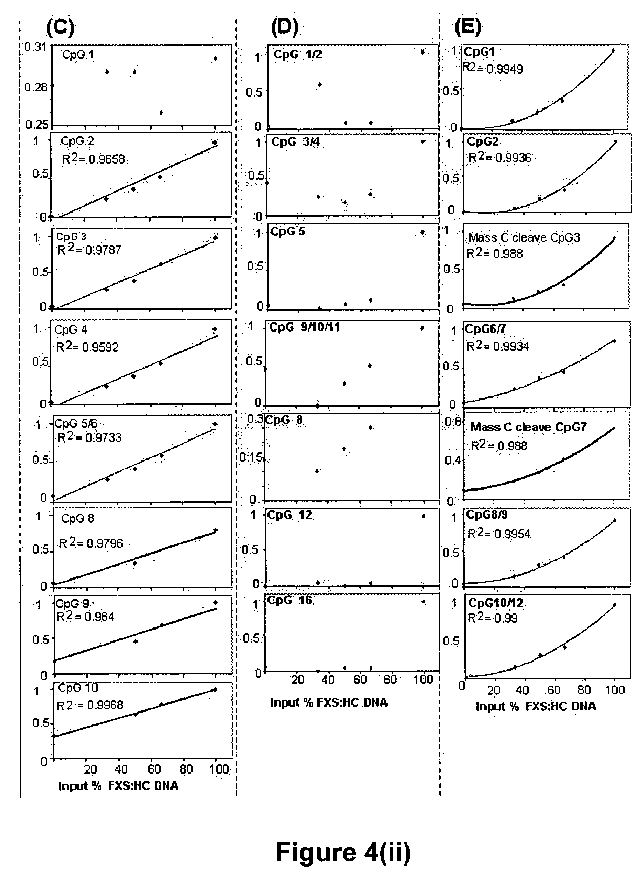

As indicated above, the sequence targeted by Amplicon 2 encompasses all FMR1 transcription start sites. The main transcription start site and the alternative Inr-like start site I are located between CpGs 11 and 12, Inr-like start site II is located between CpGs 7 and 8, and Inr-like start site 3 is located between CpGs 5 and 7 of the Amplicon 2. The Inr-like sites are conserved between mammalian species. One of the two main FMR1 regulatory transcription factor binding sites functionally influenced by methylation - USF1/2 binding site Footprint I is located at the CpG unit 6 of the Amplicon 2. Analysis of DNA from 4 healthy controls and 3 FXS individuals demonstrated that the CpGs 5 to 12 surrounding the four FMR1 transcription start sites and the USF1/2 binding site Footprint I is fully methylated in FXS, but not in healthy control DNA (Figure 3). There is also a weak methylation signal detected for CpGs 8-11 in healthy controls. However, DNA spiking experiments demonstrate that this background methylation signal of a healthy control is due to technical limitation of methylation analysis within this region (Figure 4, D).

The methylation pattern of the second most important methylation dependent FMR1 regulatory motif - the ά-PAL/NRF1 binding site Footprint IV located within the same region as the ASFMR1/FMR4 putative bi-directional promoter 2, could not be directly analyzed, as it is located between Amplicons 1 and 2 targeted regions (Figure 2). Although both PAL/NRF1 binding site Footprint IV and USF1/2 binding site Footprint I are positive *cis* elements that regulate most of the FMR1 promoter activity and FMR1 transcription, it is unknown whether they are also important in regulation of antisense FMR1 expression.

It is proposed herein that Amplicon 1 region located upstream of the CGG expansion and the CpG island, which has been termed "Fragile X-Related Epigenetic Element 1" (FREE1). It should be noted that the region covered by Amplicon 1 is the 5' portion of both ASFMR1 and FMR4 transcripts, and includes a 5' portion of the FMR1 promoter. This region is potentially bi-directionally involved in regulation of FMR1, ASFMR1 and FMR4 transcription (Figure 1B). The present invention demonstrates that within Amplicon 1 (Figure 3), only CpG1 did not reflect methylation of the classical FMR1 promoter in FXS and healthy controls. In Amplicon 1, CpG units 2 to 6 were consistently fully methylated for FXS DNA and methylated for the healthy controls (Figures 3A and B). For FXS DNA, CpGs 8 to 10 were also fully methylated, and for healthy controls they were partially methylated (Figure 3A and B and Figure 1).

In contrast to FREE1 and FREE2, other regions examined, covered by Amplicon 3 and 4, did not reflect the methylation pattern of the 'classical' CpG island in FXS patients and controls (Figure 3). These regions thus served as appropriate controls for FREE1 and FREE2 methylation mapping using MALDI-TOF MS analysis in FXS and control samples, while assisting in re-defining the 5' border of the methylated locus in DNA from FXS patients. In addition, portions of these upstream regions (particularly the Amplicon 3 CpGs 6 and 7 and Amplicon 4 CpGs 7 to 10) were largely hypomethylated in FXS samples; but hypermethylated in half of the controls. This may indicate that in FXS the methylation shifts from these upstream regions to FREE1, and this may have implications for transcription from the FMR1 locus.

For the FREE2 region covered by Amplicon 5 and located 3' of the CGG expansion, we have found that all of the CpG sites reflected methylation of the classical CpG island in FXS and controls (Figure 3). Specifically, we observed complete methylation of CpGs 1, 2, 10 to 12, and partial to full methylation of CpG units 3 to 9 in all FM cell lines from FXS patients, however in the control samples all of the CpG units within the Amplicon were largely unmethylated. It should be noted that the ATG start site for FMRP translation is located in close proximity to CpG 1 of FREE2. CpGs 3 to 12 of FREE2 are located within intron 1 of the FMR1 gene, and encompass seven putative GATA binding sites (Figures 2 and 3 and Table 5).

### EXAMPLE 2

### Determining the impact of technical variation on quantitative analysis of methylation

DNA from lymphoblasts of healthy controls with 30 CGG repeats, normal levels of FMR1 mRNA and FMRP, and DNA from lymphoblasts of FXS patient with 530 CGG, silenced FMR1 transcription and absence of FMRP were mixed at ratios of 1:0; 2:1; 1:1; 1:2; 0:1 corresponding to 0, 33.3, 50, 66.6, 100% FXS DNA in the sample. The spiked DNA samples were bisulfite converted in duplicate reactions. Each reaction was amplified with primer sets (forward and reverse primers) as listed by SEQ ID NOs:3, 4, 5, 6, 7, 8, 9, 10, 11 and 12 which corresponded to Amplicons 1 to 5, respectively. Mean methylation calls of the duplicate reactions for each sample were plotted against the expected FXS DNA content (with fully methylated FMR1 promoter). For CpG units, this provides fully quantitative analysis of FXS DNA content, it was expected that there would be a linear relationship with a positive gradient, approaching x=y. However, of the 5 Amplicons analyzed, only Amplicons 1, 2 and 5 had a consistently positive gradient for the spiked samples (Figures 4, C, D and E), and could be used to distinguish between healthy control and FXS DNA. Of these, Amplicon 1 consistently displayed the most linear relationship for 7 out of 8 CpGs, with high Pierson's correlation squared.

CpG units within Amplicon 5 also displayed high correlation between methylation output and expected FXS DNA content, however, the relationship had a consistently polynomial component, with a clear bias towards healthy control DNA amplification. This phenomenon was most extreme for Amplicon 2 which encompassed the CGG expansion, where any quantity of spiked healthy control DNA was preferentially amplified to FXS DNA. Furthermore, the reaction failure rate for Amplicon 2 was the highest of all the Amplicons examined (Figure 3). This phenomenon can be explained by CGG expansion being inhibitory to PCR amplification of target regions in its close proximity, or to the PCR products that encompass it.

### EXAMPLE 3

### Reproducibility of methylation profile

The reproducibility of the methylation profile of Amplicon 1 was examined for both inter- variation (Figure 5A) and intra-variation (Figure 5B and 5C) using at least 4 repeats per sample in the experiment.

FREE1 methylation was examined in DNA extracted from whole blood of male CGG repeat carriers, ranging from normal size alleles to full mutation. Duplicate bisulfite reactions were made from each sample, which were amplified in duplicate PCR reactions - providing a total of 4 measurements per sample for MALDI-TOF methylation analysis.

Referring to Figure 5**(A)****,** this shows the inter-run variation in the methylation output ratio for DNA from 1 healthy control (HC), 3 grey zone (GZ), 4 premutation (PM) and 2 Fragile X Syndrome (FXS) individuals. CpGs 2 to 10 could be clearly used to distinguish fully methylated FXS samples from PM, GZ and HC cell lines, all largely unmethylated. However, this was not the case for CpG 1 that showed no differential methylation between the four categories

The inter-run variance in the CGG repeat carriers, ranging from normal size alleles to full mutation, was not consistent between different units within FREE 1. The results for the CpG 10 were the most variable with mean variance of 0.14, while CpGs 1 to 6 were the most consistent, with mean variances between 0.02 and 0.05. CpG units 8 and 9 had intermediate levels of variability, with mean variances of 0.06 and 0.09 respectively

Figure 5(**B**) shows the intra-run variation in the methylation output ratio for DNA from 4 healthy controls and 3 FXS individuals. CpGs 10 and 8 were also the least consistent, with the mean variances for both being ∼0.3. CpG units 1, 5/6 and 9 had intermediate levels for the intra-run variation, with mean variance of ~0.1. Similar to inter-run variation, CpG units 1 to 4 had the most consistent intra-run variation values, with mean variances between 0.02 and 0.05.

One of the major factors that may have contributed to elevated inter and a run technical variation of CpG units 9 and 10, may have been presence of a silent peak for these units. Despite of this, Figure 4 clearly demonstrates that both CpGs 9 and 10 can be confidently used to distinguish FM DNA from FXS males from the smaller expansion carrier males.

Figure 5(**C**) is a graphical representation of the relationship between the mean methylation output across Amplicon 1 CpG 2 to 10 and the HC DNA spiked with 0, 33.3, 50, 66.6, 100% FXS DNA. The different lines represent 3 separate runs for the same samples, and demonstrate the intra-run variation in the spiked samples at different levels of methylation. The error bars represent the standard error.

### EXAMPLE 4

### Diagnosis based on methylation of CpG units in Amplicon 1

### (A) Subjects with full mutations affected with the Fragile X syndrome versus healthy controls

Using the FREE1 region, identified as providing a correlation between biologically significant results and CpG methylation, was assessed. Methylation of the FREE1 region was examined in DNA extracted from whole blood, amnyocytes, CVS and lymphoblasts of healthy controls, CGG repeat carriers ranging from normal size alleles to full mutation. It is clear from the results that methylation patterns in a variety of tissues can distinguish which individual, either male or female, has a full mutation and affected with the Fragile X Syndrome [FXS] compared to healthy individuals, GZ and PM carriers (Figures 6A, B, C, D and E).

### (B) Subjects with premutation or Gray Zone versus full mutation subjects or healthy controls with normal size CGG tract

The different methylation patterns of the FREE 1 region, can distinguish female carriers of permutation alleles from FXS females (Figure 6B). The methylation pattern can be also used to distinguished female carriers of Premutation [PM] or Gray Zone [GZ] alleles with skewed X-inactivation compared to healthy individuals with normal size CGG tract (Figure 6). The methylation pattern can be also used to distinguished subjects with abnormally skewed X-inactivation as in female CVS from normal ∼50% X-inactivation pattern found in blood of healthy control females (Figure 6F).

### EXAMPLE 5

### Distinguishing the epigenetic profile of subjects using the FMR genetic locus relative to transcription

### (A) Subjects with full mutations affected with the Fragile X Syndrome versus healthy controls

There is significant inhibition of transcription of the FMR4, ASFMR1 and FMR1 genes in FXS patients which is likely caused by the methylation of the CpG units in Amplicon 1 (FREE 1 region), and/or Amplicon 5 (FREE 2 region) in association with the FMR1 promoter. It is demonstrated here that in subjects with FM affected with FXS with no FMR1 mRNA (Figure 1I) or FMRP and significantly decreased from normal ASFMR1/FMR4 mRNA levels (Figure 1J), FREE1 CpG units 2 through to 10 were fully methylated. In these same cell lines the FREE1 and FREE2 regions and the FMR1 promoter were fully methylated. In healthy controls the FREE1 and FREE2 regions and the FMR1 promoter (Figures 3A) were unmethylated, with normal levels of FMR1 and FMR4/ASFMR1 mRNA (Figure 1C and D) and FMRP expression. Furthermore, in the cell lines for FM carriers affected with FXS, demethylation of FREE1 with 5-aza treatment was associated an increase in mRNA levels of FMR1 and ASFMR1/FMR4 (Figure 1I and J).

Therefore, the method of the present invention clearly identifies individuals with the FM of FXS. These data also indicate that methylation of the FREE1 regions is closely related to inhibition of bi-directional transcription and translation of the FMR locus required from normal neuronal development. As a consequence, this can lead to pathological conditions such as FXS, mental retardation and autism. Hence, the assay herein can be used to diagnose, make a prognosis and detect the presence or predisposition to FXS, and potentially any other neuropathological condition/s associated with elevated methylation and/or altered distribution of methylated sites in the FMR locus described herein.

### (B) Subjects with Premutation or Gray Zone versus healthy controls with normal size CGG tract

There is significant increase in the 5' FMR4/ASFMR1 mRNA which includes the FREE1 region in male subjects with PM alleles compared to HC and GZ cell lines. A significant increase in transcription was also demonstrated in the GZ compared to HC cell lines (Figure 1D). These GZ and PM cell lines have an altered FREE1 methylation pattern (Figure IE) and increased levels of FMR1 mRNA (Figure IF) compared to healthy controls. With further demethylation of FREE1 using the 5-aza treatment there was increase in FMR1 and ASFMR1/FMR4 mRNA levels (Figure 1F and J).Thus, these data indicate that altered methylation pattern within the FREE1 region in some GZ and PM may be related to increased transcription within the same region, and the FMR locus as a whole. This may lead to RNA toxicity resulting from the FMR locus, leading to pathological conditions such as FXTAS, POF, mental retardation and/or autism. Hence, the assay herein can be used as a prognostic or diagnostic measure of the presence or predisposition to FXTAS and potentially any other neuropathological condition/s associated with altered methylation and/or altered distribution of methylated sites in the FMR locus described herein, associated with GZ and/or PM alleles.

### EXAMPLE 6

### Comparison of FREE1 methylation analysis and CpG island methylation using Southern blot

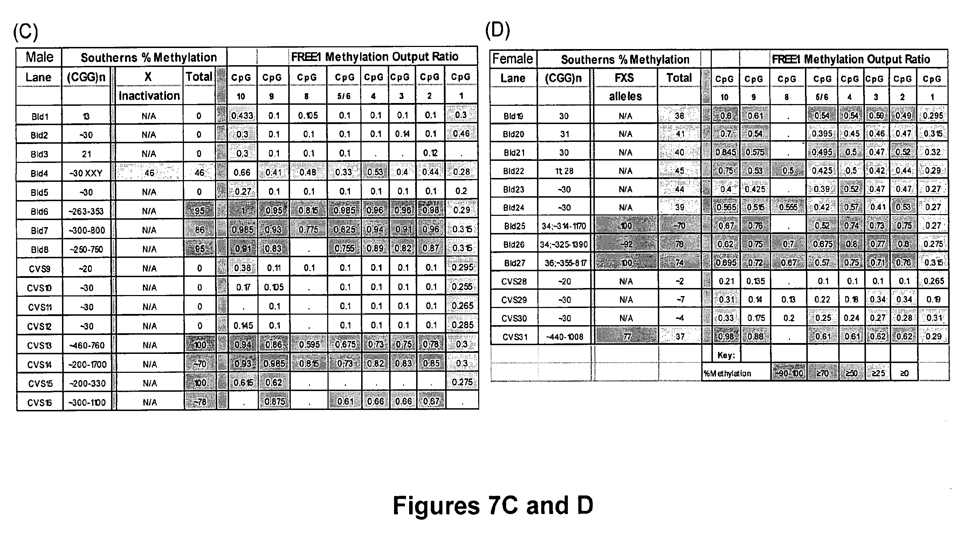

Methylation analysis was performed of the FREE1 region and the FMR1 CpG island in the same samples using MALDI-TOF and Southern blot, to determine the consistency of the FREE1 analysis and CpG island methylation in blood and CVS, from males and females. A male was included with normal size FMR1 alleles but with an additional X-chromosome, the Klinefelter's syndrome as a positive control for the FMR1 CpG island methylation analysis (Figures 7A and C lane 4).

For the female bloods, FREE1 methylation was associated with X-inactivation, as well as with hypermethylation of the CpG island of the FM alleles from FXS patients. Similar to the XXY sample, the methylation output ratios for healthy control females were between ∼0.4 and ∼0.5 for CpGs 2 to 9 (Figure 7B and D). Of these, GpGs 3 and 5/6 were most closely correlated with Southern blot methylation analysis related to X-inactivation (Figure 7B, lanes 19 to 24). For the FXS bloods, the FREE1 methylation output ratios for CpGs 2 to 9 approached 0.8, which was closely reflected by Southern blot analysis of the total methylation levels for the CpG island (Figures 7B and D, lanes 25 to 27).

Southern blot was also used to examine methylation status of only FM alleles. It was found that the values (∼90 to 100% methylated) were marginally higher than from the total methylation analysis MALDI-TOF output (∼80%) [Figures 7B and D; lanes 25 to 27). This indicated that the X-inactivation ratio marginally skewed methylation measurements for FXS alleles for both the Southern blot and MALDI-TOF analysis, when total methylation values were used in the female samples.

For the CVS of a mosaic FM female collected at 17 weeks gestation the methylation output ratio for FREE1 CpGs 9 and 10 approached 1. This was closely reflected by the Southern blot methylation values for the FM alleles only, of 77% (Figures 7B and D). However, the total methylation values for this sample using Southern blot indicated only 37% methylation. This suggested that lack of X-inactivation in this sample significantly skewed the total methylation results. Furthermore, we have found that the 3' portion of the FREE1 region closest to the CpG island behaved in a similar fashion. It had marginally lower methylation output ratios of ∼0.6 for CpGs 2, 3, 4, 5/6 (∼60% total methylation) and the 5' portion of FREE1 - CpGs 9 and 10.

This partially methylated portion of the FREE1 region in FM CVS/mosaic female is located in close proximity to the restriction site targeted by the Southern analysis. Since Southern analysis demonstrated X-inactivation values of ∼10 to ∼20% in CVS of healthy control females, it may explain why for the FM CVS female the FREE1 total methylation output of the CpGs 2, 3, 4, 5/6 and the total methylation values for Southern blot appeared to be much lower than expected, suggesting that both the 5' portion of FREE1 and the classical CpG island are affected by X-inactivation bias in CVS to a similar degree.

The Southern blot analysis of only FM alleles in the female CVS also indicated similar levels of methylation (77%) to that in CVS of FXS males (between 70 and 100%), where male and female samples were collected at the same gestational age (Figures 7A and C). This suggested that the total methylation values for the CVS females are related to incomplete methylation and chromatin spreading associated with X-inactivation up to 17 weeks of fetal development rather than incomplete methylation of the FM alleles.

Together data presented in Example 6 demonstrate that FREE1 MALDI-TOF MS analysis is highly consistent with Southern blot methylation analysis of DNA from blood and CVS in males and females, as well as from patients with X chromosome abnormalities such as the Klinefelter's syndrome.

### EXAMPLE 7

### FREE1 and FREE2 methylation on FM alleles of "high functioning" FM carrier males and Klinefelter's Syndrone affected patients

In order to determine which region is the most informative in biological settings, the spiking experiment was mimicked by analyzing the methylation patterns of FREE1 and FREE2 methylation in blood of "high functioning" male carriers of unmethylated (0% classical CpG island methylation by Southern blot) and partially methylated (∼30% classical CpG island methylation by Southern blot) FM alleles, as well as fully methylated FM alleles (100% classical CpG island methylation by Southern blot), and Klinefelter's syndrome affected individuals with normal size CGG alleles (50% classical CpG island methylation by Southern blot). It was found that the FREE1 could be used to distinguish unmethylated FM carriers from ∼30%, ∼50% and ∼100% methylated alleles, whereas FREE2 could not distinguish between unmethylated and 30% methylated FM samples (Figures 8A and B). Methylation of both FREE1 and FREE2 could be used to distinguish Klinefelter's patients from full mutation carriers with fully methylated and unmethylated FMR1 alleles.

### EXAMPLE 8

### Clinical applications of FREE1 and FREE1 methylation analyses

### Methylation status of FREE1 and FREE2 in "high functioning" males and FM carrier females

To determine which region is the most informative in biological settings, methylation of FREE1 and FREE2 was examined in blood samples from 21 "high functioning" males including mosaic individuals, and compared the results with those from Southern blot methylation of the classical CpG island and with FMRP expression. Here a complete data set was used for 15 individuals for the analysis of the relationship between FMRP levels and methylation. For correlation between FREE1 and FREE2 methylation and Southern blot analysis a two additional blood samples were included from FXS individuals with low IQ (∼47) and no FMRP expression, making the total of samples analyzed to 23.

The mean methylation across FREE1 (CpG units 2 to 10) was most closely correlated with the number of FMRP positive lymphocytes of all regions examined (Figure 9A) [mean methylation across FREE1 R-0.62; p=0.01; FREE2 R=-0.55; p=0.03 and Southern blot methylation R=-0.59; p=0.01). Southern blot methylation was positively correlated with both FREE1 (R=0.97; p<0.00001) and FREE2 (R=0.93; p<0.00001). Mean FREE1 and FREE2 methylation values were also closely related to each other (R=0.97; p<0.00001).

It is also of interest that in three FM carriers with high standardized 1 Q (65-76) and mild to moderate deficits in FMRP, the methylation of both FREE1 and FREE2 was not related to elevated FMR1 mRNA levels (Figure 9A). This indicates that mean FREE1 and FREE2 methylation analyses do not reflect RNA toxicity associated with the expanded alleles, while being specific for deficits in FMRP, and thus FXS methylated "classical" CpG island, both FREE1 and FREE2 were methylated at a low level.

To determine which region is the most informative in female samples, the FREE1 and FREE2 analysis was performed in 12 FM allele carriers with variable FMR1 activation ratios and 11 healthy controls. It was found that the FMR1 activation ratio determined by Southern blot was inversely correlated with methylation status of both FREE1 (R=-0.93; p<0.0001) and FREE2 (R=-0.95; p<0.0001) [Figure 9B]. As an internal control, Nrul methylation was measured using Southern blot in nine control females that showed 0.39 +/- 0.045 (mean +/- STDEV) X-inactivation. This was consistent with FREE1 analysis (0.43 +/- 0.04), whereas FREE2 analysis showed much lower X-inactivation values (0.25 +/- 0.025) in female controls (Figure 9B).

### EXAMPLE 9

### FREE1 methylation analysis in male and female clinical samples with the full range of CGG expansions

Methylation analysis was performed on the FREE1 region (as it had the largest number of informative sites) for the larger sample set composed of DNA from 49 controls, 18 GZ, 22 PM carriers and 22 (clinically affected) FXS subjects, in males and females. The FREE1 region methylation was examined in duplicate bisulfite reactions per sample, each amplified with a single PCR reaction. Methylation output for each sample was expressed as a mean of two technical replicates, provided that the duplicate measurements were within 35% of their mean. Based on 10% technical variance of FREE1 MALDI-TOF MS any values of 0.1 or less, were considered as 0.1 (Figures 6A, B and C).

It was found that in male blood and CVS samples, FREE1 CpG units 2 to 10 were consistently hypermethylated in FXS subjects (∼70 to 100%) but unmethylated in controls, GZ and PM allele carriers. Hypomethylation in healthy control CVS was mirrored in a parallel amniocyte sample. In FM alleles of females with clinical FXS FREE1 CpG units 2 to 9 were also consistently hypermethylated (methylation output ratios of ∼0.8 to 1), whereas in healthy control females with normal size alleles the methylation output ratio approached 0.5 in blood. In female blood there were also no significant differences in FREE1 CpG units 2 to 10 methylation between GZ and PM groups (55 to ∼130 repeats) with uncharacterized phenotype. However, there was a significant increase in methylation of CpG units 1, 2, 3, 5/6 and 9 in the females with GZ/PM allele carriers compared to healthy controls, likely to be due to skewed X-inactivation (Figure 6B).

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations of any two or more of said steps or features.

**TABLE 4**

| ***Prominent regulatory motif locations and methylation sensitive restriction sites within the classical* FMR1 *CpG island (Amplicon 2), FREE1 (Amplicon 1) and FREE2 (Amplicon 5) regions*** | | | |
|---|---|---|---|
| **TRANSCRIPTION FACTOR SITES/POTENTIAL REGULATORY MOTIFS:** | **SEQUENCE**: | **AMPLICON:** | **CpG unit LOCATION:** |
| GATA1/2-SRY-Ik2-c-Ets | ACTGGGATAACCGG | 1 | CpG 3 and CpG2 |
| | ATGCATTTG | | |
| | ATTTCCCACGCCACTG | | |
| GATA2 | ATCCCAGAGA | 1 | Between CpG 4 and CpG 5/6 |
| Putative SRY binding sites | CCGGACCAAA | 1 | Between CpG 5/6 and CpG 8 |
| (CAAAC)n repetitive element | CCAAACCAAA | | |
| | CCAAACCAAA CCAAACC | | |
| Foot print IV-ά-PAL/NRF1 binding site | CGCGCATGCGC | Between 1 and 2 | N/A |
| | TCGCGA | Between 1 and 2 | N/A - detected using Southern Blot analysis |
| Eagl | CGGCCG | Between 1 and 2 | N/A |
| Foot print III - Spl binding site | GAGGGCG | 2 | CpG 1 |
| Foot print II - AP2 H4tfl/Spl binding site | CGCGGGGGGA | 2 | CpGs 3/4 |
| Foot print I - USF1/2 binding site | TCACG | 2 | CpG 6 |
| Zeste site | CGCTCA | 2 | CpG 12 |
| GATA1/GATA2 | GAAGATGGAG | 5 | 5' to CpG 1 |
| GATA1/GATA2/GATA3 | GCCCCATCTTCG | 5 | CpG 3 |
| GATA1/GATA2 | CGGGATGTTG | 5 | CpG 12 |

**TABLE 5**

| ***Sequence analysis for orthologous conservation and putative transcription factor binding sites in the sense and antisense direction for FREE1 and FREE2 regions*** | | | | | | | |
|---|---|---|---|---|---|---|---|
| | | **FREE1** | | | **FREE2** | | |
| **Inter-species conservation - % Homology to human.** | | Pan troglodytes (Common chimp) - 90% | | | Macaca mulatta (Rhesus Macaque)- 93%; Sus Scrofa (Wild boar) - 91%; Canis familiaris (dog) - 90%; Bos Taurus (cattle) - 88%; Mus musculus (house mouse) - 86%; Rattus norvegicus (Norway rat) - 86%; Monodelphis domestics (Gray short tailed opossum) - 95%. | | |

| | | **FREE1** | | | **FREE2** | | |
|---|---|---|---|---|---|---|---|
| | | **Putative high scoring transcription factor (TFS) binding sites** | | | **Putative high scoring transcription factor (TFS) binding sites** | | |
| **Leading strand** | **TFS total #** | 23 | | | 7 | | |
| | **TFS ID** | ***SRY*** | ***GATA1*** | ***GATA2*** | ***GATA1*** | ***GATA2*** | ***GATA3*** |
| | **Specific site #** | 13 | 2 | 2 | 3 | 3 | 1 |
| **Lagging strand** | **TFS total #** | 23 | | | 7 | | |
| | **TFS ID** | ***SRY*** | ***GATA1*** | ***GATA2*** | ***GATA1*** | ***GATA2*** | ***GATA3*** |
| | **Specific site #** | 11 | 3 | 3 | 3 | 3 | 1 |

### BIBLIOGRAPHY

Allingham-Hawkins et al, Am JMed Genet 83(4):322-325, 1999
Bodega et al, Hum Reprod 21(4):952-957, 2006
Chiurazzi et al, Hum Mol Genet 7(1):109113, 1998
Godler et al, BMC Clin Pathol 9:5, 2009
Coulam, Fertil Steril 38(6):645-655, 1982
Dahl et al, Clin Chem 53(4):790-793, 2007
de Vries et al, Am J Hum Genet. 58:1025-1032, 1996
Fahy et al, PCR Methods Appl. 1(1):25-33, 1991
Fleige and Pfaffl, Mol Aspects Med 27(2-3):126-139, 2006
Francis et al, Mol Diagn 5(3):221-225, 2000
Fu et al, Cell 67(6):1047-1058, 1991
Gitan et al, Genome Res. 12(1):158-164, 2002
Godler et al, Hum Mol. Genet. 10:[Epub ahed of print] doi:10.1093/hmg/ddq1037
Hagerman et al, Neurology 57(1):127-130, 2001
Hartmer, Storm et al, Nucleic Acids Res 31(9):e47, 2003
Heinemeyer et al, Nucleic Acids Res 26:362-367, 1998
Irwin et al, Cereb Cortex 10(10):1038-1044, 2000
Irizarry et al, Nature Genetics 41(2):178-186, 2009
Jacquemont et al, Am JMent Retard 109(2):154-164, 2004
Jacquemont et al, JMed Genet 42(2):e14, 2005
Jin and Warren, Hum. Mol. Genet 9(6):901-908, 2000
Jin et al, Neuron 39(5):739-747, 2003
Kenneson et al, Hum Mol Genet 10(14):14491454, 2001
Khalil et al, PLoS ONE 3(1):e1486, 2008
Khaniani et al, Mol Cytogenet 1(1):5, 2008
Kumari and Usdin, J Biol Chem 276(6):4357-4364, 2001
Ladd et al, Hum Mol Genet 16(24):3174-3187, 2007
Loesch et al, Clin Genet 67(S):412-417, 2005
Loesch et al, J Med Genet 44(3):200-204, 2007
Mitchell et al, Clin Genet 67(1):38-46, 2005
Nolin et al, Am JHum Genet 72(2):454-464, 2003
Nygren et al, Nucleic Acids Res. 33(14):e128, 2005
Pieretti et al, Cell 66(4):817-822, 1991
Pietrobono et al, Nucleic Acids Res 30(14):3278-3285, 2002
Pietrobono et al, Hum Mol Genet 14(2):267-277, 2005
Piertrobono et al, Nucleic Acids Res 30:3278-3285, 2009
Rein et al, Nucleic Acids Res. 26:2255, 1998
Sullivan et al, Hum Reprod 20(2):402-412, 2005
Tassone et al, Am J Med Genet, 97:195-203, 2000
Tassone et al, J. Mol. Diagn 10:43-49, 2008
Terracciano, et al, Am JMed Genet C Semin Med Genet 137C(1):32-37, 2005
Tost et al, Nucleic Acids Res 31(9):e50, 2003
Verkerk et al, Cell 65(5):905-914, 1991
Wojdacz et al, Nucleic Acids Res. 35(6):e41, 2007
Yegnasubramanian et al, Nucleic Acids Res. 34(3):e19, 2006
Yu et al, Science 252(5010):1179-1181, 1991

## Claims

1. An *in vitro* method for identifying a pathological condition associated with the FMR1, FMR4 and/or ASFMR1 genes in a mammalian subject including a human, said method comprising screening a cell from said subject for a change relative to a control in the extent of methylation within a region of the FMR genetic locus selected from:
i. Fragile X-related Epigenetic Element 1 (FREE 1) comprising the nucleotide sequence set forth in SEQ ID NO: 16 or a homolog thereof defined by having at least 80% nucleotide sequence identity to SEQ ID NO: 16 or which hybridizes to SEQ ID NO: 16 or its complementary form under medium stringency conditions; and
ii. Fragile X-related Epigenetic Element 2 (FREE2) comprising the nucleotide sequence set forth in SEQ ID NO: 17 or a homolog thereof defined by having at least 80% nucleotide sequence identity to SEQ ID NO: 17 or which hybridizes to SEQ ID NO: 17 or its complementary form under medium stringency conditions,
wherein a change in extent of methylation relative to a control is indicative of the presence of the pathological condition or a propensity to develop same.

2. The method of claim 1 wherein the change in extent of methylation relative to a control is an increase in methylation.

3. An *in vitro* method for screening for an agent which modulates methylation of an FMR genetic locus in a mammalian cell including a human cell, said method comprising screening for a change relative to a control in the extent of methylation within a region selected from the regions defined in claim 1 in the presence or absence of an agent to be tested wherein the agent is selected if it induces a change in extent of methylation.

4. An *in vitro* method for identifying in a genome of a mammalian cell including a human cell, a pathological condition associated with the FMR1, FMR4 and/or ASFMR1 genes said method comprising extracting genomic DNA from said cell and subjecting the DNA to an amplication reaction using primers selective of a region of the FMR genetic locus selected from the regions defined in claim 1, and subjecting the amplified DNA to a methylation assay to determine the extent of methylation of the DNA wherein a change in extent of methylation relative to a control is indicative of the presence of the pathological condition or propensity to develop same.

5. The method of claim 4 wherein the change in extent of methylation relative to a control is an increase in methylation.

6. The method or use of Claim 1, 2, 4 or 5 wherein the pathological condition is (i) a neurodevelopmental or neurodegenerative condition, or (ii) Fragile X-associated primary ovarian insufficiency (FXPOI).

7. The method or use of Claim 6, wherein the neurodevelopmental or neurodegenerative condition is selected from Fragile X Syndrome (FXS), Fragile X-associated Tremor Ataxia Syndrome (FXTAS), autism, and mental retardation.

8. The method or use of Claim 1 or 4 wherein the cell is a cultured or uncultured Chorionic Villi Sample (CVS) cell, a lymphoblast cell, a blood cell, buccal cell, or an EBV lymphoblast transformed cell line.

9. The method or use of Claim 1 or 4 wherein the epigenetic assay is conducted in conjunction with an assay which determines the length of (CGG)ₙ expansion leading to a (CGG)ₙ expansion pathology selected from a GZ pathology, a PM pathology and a FM pathology.

10. Use of primers which amplify regions of the FMR genetic locus selected from:
i. Fragile X-related Epigenetic Element 1 (FREE 1) comprising the nucleotide sequence set forth in SEQ ID NO: 16 or a homolog thereof defined by having at least 80% nucleotide sequence identity to SEQ ID NO: 16; and
ii. Fragile X-related Epigenetic Element 2 (FREE2) comprising the nucleotide sequence set forth in SEQ ID NO: 17 or a homolog thereof defined by having at least 80% nucleotide sequence identity to SEQ ID NO: 17,
in the manufacture of a diagnostic kit or device to detect methylation of the FMR locus-associated with a pathological condition associated with the FMR1, FMR4 and/or ASFMR1 genes.

11. A kit for use in the method of Claim 1 or 4 comprising primers which amplify a region within the FMR genetic locus selected from:
i. Fragile X-related Epigenetic Element 1 (FREE 1) comprising the nucleotide sequence set forth in SEQ ID NO: 16 or a homolog thereof defined by having at least 80% nucleotide sequence identity to SEQ ID NO: 16; and
ii. Fragile X-related Epigenetic Element 2 (FREE2) comprising the nucleotide sequence set forth in SEQ ID NO: 17 or a homolog thereof defined by having at least 80% nucleotide sequence identity to SEQ ID NO: 17.

12. The use or kit of either of claim 10 or 11 wherein said primers are selected from the list consisting of SEQ ID NOs:3 and 4; and SEQ ID NOs:11 and 12.

13. A computer program product for operating a computer such that said computer can assess progression of a pathological condition associated with the FMR locus in a subject, the product comprising:
(1) means to assign index values to one or more of:
(a) change in methylation relative to a control at sites within FREE1 comprising the nucleotide sequence set forth in SEQ ID NO:16 or a homolog thereof defined by having at least 80% nucleotide sequence identity to SEQ ID NO:16 or which hybridizes to SEQ ID NO:16 or its complementary form under medium stringency conditions upstream of the FMR1 promoter;
(b) change of methylation relative to a control at sites within FREE2 comprising the nucleotide sequence set forth in SEQ ID NO:17 or a homolog thereof defined by having at least 80% nucleotide sequence identity to SEQ ID NO: 17 or which hybridizes to SEQ ID NO:17 or its complementary form under medium stringency conditions upstream of the FMR1 promoter;
(c) length of (CGG)ₙ expansion within the FMR genetic locus when considered in combination with (a) and/or (b);;
(2) means to convert index value to a code; and
(3) means to store the code in a computer readable medium.

14. A computer for assessing an association between extent of methylation within the FMR locus, the FMR locus and progression of a disease condition wherein the computer comprises:
(1) a machine-readable data storage medium comprising a data storage material encoded with machine-readable data, wherein the machine-readable data comprise index values associated with the features of one or more of:
a) change of methylation relative to a control at sites within FREE1 comprising the nucleotide sequence set forth in SEQ ID NO: 16 or a homolog thereof defined by having at least 80% nucleotide sequence identity to SEQ ID NO: 16 or which hybridizes to SEQ ID NO: 16 or its complementary form under medium stringency conditions upstream of the FMR1 promoter;
(b) change of methylation or other epigenetic modification relative to a control at sites within FREE2 comprising the nucleotide sequence set forth in SEQ ID NO: 17 or a homolog thereof defined by having at least 80% nucleotide sequence identity to SEQ ID NO: 17 or which hybridizes to SEQ ID NO:17 or its complementary form under medium stringency conditions upstream of the FMR1 promoter;
(c) length of (CGG)ₙ expansion within the FMR genetic locus when considered in combination with (a) and/or (b);
(2) means to convert index value to a code; and
(3) means to store the code in a computer readable medium.

15. An *in vitro* method of identifying an epigenetic profile in a population of subjects indicative of a pathological condition associated with the FMR1, FMR4 and/or ASFMR1 genes, said method comprising screening for a change relative to a control in a statistically significant number of subjects the extent of methylation within a region of the FMR genetic locus selected from the regions defined in claim 1, wherein a change in extent of methylation is indicative of the presence of the pathological condition or a propensity to develop same in the population.

16. The method of claim 15 wherein the change in extent of methylation is an increase in methylation.

## Patentansprüche

1. Ein *In vitro*-Verfahren zum Identifizieren von einem pathologischen Zustand assoziiert mit FMR1, FMR4 und/oder ASFMR1 Genen in einem Säugetier einschließlich eines Menschen, wobei das besagte Verfahren ein Zell-Screening von dem besagten Subjekt umfasst für eine Änderung relativ zu einer Kontrolle in dem Ausmaß von der Methylierung innerhalb einer Region von dem FMR genetischen Ort, ausgewählt aus:
i. Fragiles X-bezogenes Epigenetisches Element 1 (FREE 1), umfassend die angegebene Nukleotidsequenz in SEQ ID NO: 16 oder ein Homolog davon, definiert durch mindestens 80 % Nukleotid-Sequenzidentität zu SEQ ID NO: 16 oder welche SEQ ID NO: 16 hybridisiert oder ihrer komplementären Form unter Bedingungen mittlerer Stringenz; und
ii. Fragiles X-bezogenes Epigenetisches Element 2 (FREE 2), umfassend die angegebene Nukleotidsequenz in SEQ ID NO: 17 oder ein Homolog davon, definiert durch mindestens 80 % Nukleotid-Sequenzidentität zu SEQ ID NO: 17 oder welche SEQ ID NO: 17 hybridisiert oder ihrer komplementären Form unter Bedingungen mittlerer Stringenz,
wobei eine Änderung im Ausmaß von der Methylierung relativ zu einer Kontrolle bezeichnend ist von der Anwesenheit von dem pathologischen Zustand oder eine Neigung den selbigen zu entwickeln.

2. Das Verfahren nach Anspruch 1, wobei die Änderung in dem Ausmaß der Methylierung relativ zu einer Kontrolle ein Anstieg der Methylierung ist.

3. Ein *In vitro*-Verfahren für das Screening für ein Mittel, welches die Methylierung von einem FMR genetischen Ort in einer Säugetierzelle einschließlich einer Menschenzelle moduliert, wobei besagte Methode ein Screening für eine Änderung relativ zu einer Kontrolle in dem Ausmaß der Methylierung innerhalb einer Region ausgewählt von den Regionen definiert in Anspruch 1 in der Anwesenheit oder Abwesenheit von einem Mittel, welches getestet wird, umfasst, wobei das Mittel ausgewählt wird, ob es einen Wechsel im Ausmaß der Methylierung veranlasst.

4. Ein *In vitro*-Verfahren für die Identifizierung in einem Genom einer Säugetierzelle, einschließlich einer menschlichen Zelle, einen pathologischen Zustand assoziiert mit FMR1, FMR4 und/oder ASFMR1 Genen, wobei besagtes Verfahren Extrahieren von der genomischen DNA von besagter Zelle und Subjektiveren von der DNA zu einer Amplifikationreaktion unter Verwendung von ausgewählten Primern von einer Region von dem FMR genetischen Ort ausgewählt von den Regionen definiert in Anspruch 1 umfasst, und Subjektivieren von der amplifierten DNA zu einem Methylierungs-Assay, um das Ausmaß von der Methylierung von der DNA zu bestimmen, wobei eine Änderung in dem Ausmaß von der Methylierung relativ zu einer Kontrolle bezeichnend ist für die Anwesenheit von dem pathologischen Zustand oder einer Neigung den selbigen zu entwickeln.

5. Das Verfahren nach Anspruch 4, wobei die Änderung im Ausmaß von der Methylierung relativ zu einer Kontrolle ein Anstieg der Methylierung ist.

6. Das Verfahren oder Verwendung nach Anspruch 1, 2, 4 oder 5, wobei der pathologische Zustand (i) ein Neuroentwicklungs- oder Neurodegenerativer Zustand, oder (ii) Fragile X-assoziierte primäre Ovarialinsuffizienz (FXPOI) ist.

7. Das Verfahren oder Verwendung nach Anspruch 6, wobei der Neuroentwicklungs oder Neurodegenerativer Zustand ausgewählt ist von Fragiles X -Syndrom (FXS), Fragiles X-assoziiertes Tremor Ataxia Syndrom (FXTAS), Autismus, und mentale Behinderung.

8. Das Verfahren oder Verwendung nach Anspruch 1 oder 4, wobei die Zelle eine kultivierte oder unkultivierte Chorionix Villi Sample (CVS) Zelle ist, eine Lymphoblasten-Zelle, eine Blutzelle, eine Buccal-Zelle, oder eine EBV-Lymphoblasten-Zellelinie ist.

9. Das Verfahren oder Verwendung nach Anspruch 1 oder 4, wobei die epigenetische Probe in Verbindung mit einer Probe durchgeführt wird, welcher die Länge von (CGG)ₙ Expansion bestimmt, führend zu einer (CGG)ₙ Expansionspathologie ausgewählt von einer GZ Pathologie, einer PM Pathologie und einer FM Pathologie.

10. Verwendung von Primern, welche die Regionen von dem FMR genetischen Ort verstärken, ausgewählt von:
i. Fragiles X-bezogenes Epigenetisches Element 1 (FREE 1), umfassend die angegebene Nucleotidsequenz in SEQ ID NO: 16 oder ein Homolog davon, definiert durch mindestens 80 % Nukleotid-Sequenzidentität zu SEQ ID NO: 16; und
ii. Fragiles X-bezogenes Epigenetisches Element 2 (FREE 2), umfassend die angegebene Nucleotidsequenz in SEQ ID NO: 17 oder ein Homolog davon, definiert durch mindestens 80 % Nukleotid-Sequenzidentität zu SEQ ID NO: 17,
bei der Herstellung eines diagnostischen Kits oder Anordnung, um die Methylierung des FMR Ortes-assoziiert mit einem pathologischen Zustand mit dem FMR1, FMR4 und/oder ASFMR1 Genen zu erfassen.

11. Ein Kit zur Verwendung in dem Verfahren nach Anspruch 1 oder 4, umfassend Primer, die eine Region innerhalb des FMR genetischen Ortes amplifizieren, ausgewählt aus:
i. Fragiles X-bezogenes Epigenetisches Element 1 (FREE 1), umfassend die angegebene Nucleotidsequenz in SEQ ID NO: 16 oder ein Homolog davon, definiert durch mindestens 80 % Nukleotid-Sequenzidentität zu SEQ ID NO: 16; und
ii. Fragiles X-bezogenes Epigenetisches Element 2 (FREE 2), umfassend die angegebene Nucleotidsequenz in SEQ ID NO: 17 oder ein Homolog davon, definiert durch mindestens 80 % Nukleotid-Sequenzidentität zu SEQ ID NO: 17.

12. Die Verwendung oder das Kit nach einem der Ansprüche 10 oder 11, wobei die Primer aus der Liste ausgewählt sind, bestehend aus SEQ ID NOs: 3 und 4; und SEQ ID NOs: I1 und 12.

13. Ein Computerprogrammprodukt für die Betreibung von einem Computer, so dass besagter Computer einen Fortschritt von einem pathologischen Zustand beurteilen kann, assoziiert mit dem FMR Ort in einem Subjekt, das Produkt umfasst:
(1) Mittel, um einen Indexwert zu einem oder mehren zuzuordnen:
(a) Wechsel in der Methylierung relativ zu einer Kontrolle bei den Plätzen innerhalb FREE 1 umfassend die angegebene Nucleotidsequenz in SEQ ID NO: 16 oder ein Homolog davon, definiert durch mindestens 80 % Nukleotid-Sequenzidentität zu SEQ ID NO: 16 oder welche SEQ ID NO: 16 hybridisiert oder ihrer komplementären Form unter Bedingungen mittlerer Stringenz ansteigend von dem FMR1 Promoter;
(b) Wechsel in der Methylierung relativ zu einer Kontrolle bei den Plätzen innerhalb FREE 2 umfassend die angegebene Nucleotidsequenz in SEQ ID NO: 17 oder ein Homolog davon, definiert durch mindestens 80 % Nukleotid-Sequenzidentität zu SEQ ID NO: 17 oder welche SEQ ID NO: 17 hybridisiert oder ihrer komplementären Form unter Bedingungen mittlerer Stringenz ansteigend von dem FMR1 Promoter;
(c) Länge von (CGG)ₙ Ausweitung innerhalb des FMR genetischen Ortes, wenn in Kombination mit (a) und/oder (b) berücksichtigt;
(2) Mittel, um den Indexwert in einen Code umzuwandeln; und
(3) Mittel, um den Code in einem computerlesbaren Medium zu speichern.

14. Ein Computer zur Beurteilung einer Assoziation zwischen dem Ausmaß der Methylierung innerhalb des FMR Ortes, des FMR Ortes und dem Fortschritt von einem Zustand einer Krankheit, wobei der Computer umfasst:
(1) ein maschinenlesbares Datenspeichermedium umfassend ein Datenspeichermaterial mit maschinenlesbaren Daten, wobei die maschinenlesbaren Daten Indexwerte umfassen, assoziiert mit den Merkmalen von einem oder mehreren von:
(a) Wechsel in der Methylierung relativ zu einer Kontrolle bei den Plätzen innerhalb FREE 1 umfassend die angegebene Nucleotidsequenz in SEQ ID NO: 16 oder ein Homolog davon, definiert durch mindestens 80 % Nukleotid-Sequenzidentität zu SEQ ID NO: 16 oder welche SEQ ID NO: 16 hybridisiert oder ihrer komplementären Form unter Bedingungen mittlerer Stringenz ansteigend von dem FMR1 Promoter;
(b) Wechsel in der Methylierung relativ zu einer Kontrolle bei den Plätzen innerhalb FREE 2 umfassend die angegebene Nucleotidsequenz in SEQ ID NO: 17 oder ein Homolog davon, definiert durch mindestens 80 % Nukleotid-Sequenzidentität zu SEQ ID NO: 17 oder welche SEQ ID NO: 17 hybridisiert oder ihrer komplementären Form unter Bedingungen mittlerer Stringenz ansteigend von dem FMR1 Promoter;
(c) Länge von (CGG)ₙ Ausweitung innerhalb des FMR genetischen Ortes, wenn in Kombination mit (a) und/oder (b) berücksichtigt;
(2) Mittel, um den Indexwert in einen Code umzuwandeln; und
(3) Mittel, um den Code in einem computerlesbaren Medium zu speichern.

15. Ein In vitro Verfahren zum Identifizieren von einem epigenetischen Profil in einer Population von Subjekten bezeichnet von einem pathologischen Zustand assoziiert mit FMR1, FMR4 und/oder ASFMR1 Genen, wobei besagtes Verfahren ein Screening für einen Änderung relativ zu einer Kontrolle in einer statistisch signifikanten Nummer von Subjekten das Ausmaß von der Methylierung innerhalb einer Region von dem FMR genetischen Ort ausgewählt von den Regionen definiert in Anspruch 1 umfasst, wobei ein Wechsel im Ausmaß von der Methylierung bezeichnend ist für die Anwesenheit von dem pathologischen Zustand oder einer Neigung den selbigen in der Population zu entwickeln.

16. Das Verfahren nach Anspruch 15, wobei die Änderung in dem Ausmaß von der Methylierung ein Anstieg in der Methylierung ist.

## Revendications

1. Procédé *in vitro* d'identification d'un état pathologique associé aux gènes FMR1, FMR4 et/ou ASFMR1 chez un sujet mammifère, y compris un être humain, ledit procédé comprenant la recherche, dans une cellule dudit sujet, d'une modification par rapport à un témoin de l'étendue de la méthylation à l'intérieur d'une région du locus génétique FMR sélectionnée parmi :
i. un élément épigénétique lié à l'X fragile 1 (FREE 1) comprenant la séquence nucléotidique présentée dans la SEQ ID NO: 16 ou une homologue de celle-ci définie comme ayant une identité de séquence nucléotidique d'au moins 80 % avec la SEQ ID NO: 16 ou qui s'hybride à la SEQ ID NO: 16 ou à sa forme complémentaire dans des conditions de stringence moyenne ; et
ii. un élément épigénétique lié à l'X fragile 2 (FREE 2) comprenant la séquence nucléotidique présentée dans la SEQ ID NO: 17 ou une homologue de celle-ci définie comme ayant une identité de séquence nucléotidique d'au moins 80 % avec la SEQ ID NO:17 ou qui s'hybride à la SEQ ID NO:17 ou à sa forme complémentaire dans des conditions de stringence moyenne,
dans lequel une modification de l'étendue de la méthylation par rapport à un témoin est indicative de la présence de l'état pathologique ou d'une propension à développer celui-ci.

2. Procédé selon la revendication 1, dans lequel la modification de l'étendue de la méthylation par rapport à un témoin est une augmentation de la méthylation.

3. Procédé *in vitro* de recherche d'un agent qui module la méthylation d'un locus génétique FMR dans une cellule de mammifère, y compris une cellule humaine, ledit procédé comprenant la recherche d'une modification par rapport à un témoin de l'étendue de la méthylation dans une région sélectionnée parmi les régions définies dans la revendication 1 en présence ou en l'absence d'un agent à tester, dans lequel l'agent est sélectionné s'il induit une modification de l'étendue de la méthylation.

4. Procédé *in vitro* pour identifier dans un génome une cellule de mammifère, y compris une cellule humaine, un état pathologique associé aux gènes FMR1, FMR4 et/ou ASFMR1, ledit procédé comprenant l'extraction de l'ADN génomique de ladite cellule et la soumission de l'ADN à une réaction d'amplification en utilisant des amorces sélectives d'une région du locus génétique FMR sélectionnée parmi les régions définies dans la revendication 1, puis la soumission de l'ADN amplifié à un dosage de la méthylation pour déterminer le degré de méthylation de l'ADN, dans lequel une modification de l'étendue de la méthylation par rapport à un témoin est indicative de la présence de l'état pathologique ou d'une propension à développer celui-ci.

5. Procédé selon la revendication 4, dans lequel la modification de l'étendue de la méthylation par rapport à un témoin est une augmentation de la méthylation.

6. Procédé ou utilisation selon la revendication 1, 2, 4 ou 5, dans lequel l'état pathologique est (i) une pathologie neurodégénérative ou neurodéveloppementale, ou (ii) une insuffisance ovarienne précoce liée à l'X fragile (FXPOI).

7. Procédé ou utilisation selon la revendication 6, dans lequel la pathologie neurodégénérative ou neurodéveloppementale est sélectionnée parmi le syndrome de l'X fragile (FXS), le syndrome de tremblement-ataxie lié à l'X fragile (FXTAS), l'autisme et le retard mental.

8. Procédé ou utilisation selon la revendication 1 ou 4, dans lequel la cellule est une cellule cultivée ou non cultivée d'un échantillon de villosités choriales (CVS), une cellule lymphoblastique, une cellule sanguine, une cellule buccale, ou une lignée cellulaire lymphoblastique transformée par le virus d'Epstein-Barr (EBV).

9. Procédé ou utilisation selon la revendication 1 ou 4, dans lequel le dosage épigénétique est effectué conjointement à un dosage qui détermine la longueur de l'expansion de (CGG)ₙ conduisant à une pathologie de l'expansion de (CGG)ₙ sélectionnée parmi une pathologie GZ, une pathologie PM et une pathologie FM.

10. Utilisation d'amorces qui amplifient les régions du locus génétique FMR sélectionnées parmi :
i. un élément épigénétique lié à l'X fragile 1 (FREE 1) comprenant la séquence nucléotidique présentée dans la SEQ ID NO: 16 ou une homologue de celle-ci définie comme ayant une identité de séquence nucléotidique d'au moins 80 % avec la SEQ ID NO : 16 ; et
ii. Un élément épigénétique lié à l'X fragile 2 (FREE 2) comprenant la séquence nucléotidique présentée dans la SEQ ID NO: 17 ou une homologue de celle-ci définie comme ayant une identité de séquence nucléotidique d'au moins 80 % avec la SEQ ID NO : 17,
dans la fabrication d'une trousse de diagnostic ou d'un dispositif pour détecter la méthylation du locus FMR associé à un état pathologique associé aux gènes FMR1, FMR4 et/ou ASFMR1.

11. Trousse destinée à être utilisée dans le procédé selon la revendication 1 ou 4 comprenant des amorces qui amplifient une région à l'intérieur du locus génétique FMR sélectionnée parmi :
i. un élément épigénétique lié à l'X fragile 1 (FREE 1) comprenant la séquence nucléotidique présentée dans la SEQ ID NO: 16 ou une homologue de celle-ci définie comme ayant une identité de séquence nucléotidique d'au moins 80 % avec la SEQ ID NO : 16 ; et
ii. un élément épigénétique lié à l'X fragile 2 (FREE 2) comprenant la séquence nucléotidique présentée dans la SEQ ID NO: 17 ou une homologue de celle-ci définie comme ayant une identité de séquence nucléotidique d'au moins 80 % avec la SEQ ID NO : 17.

12. Utilisation ou trousse selon l'une des revendications 10 ou 11, dans laquelle lesdites amorces sont sélectionnées dans la liste consistant en SEQ ID NO : 3 et 4, et SEQ ID NO : 11 et 12.

13. Produit programme informatique pour exploiter un ordinateur de telle sorte que ledit ordinateur puisse évaluer la progression d'un état pathologique associé au locus FMR chez un sujet, ledit produit comprenant :
(1) des moyens pour assigner des valeurs d'indice à un ou plusieurs de ce qui suit :
(a) modification de la méthylation par rapport à un témoin au niveau de sites à l'intérieur de FREE1 comprenant la séquence nucléotidique présentée dans la SEQ ID NO: 16 ou une homologue de celle-ci définie comme ayant une identité de séquence nucléotidique d'au moins 80 % avec la SEQ ID NO : 16 ou qui s'hybride à la SEQ ID NO:16 ou à sa forme complémentaire dans des conditions de stringence moyenne en amont du promoteur FMR1 ;
(b) modification de la méthylation par rapport à un témoin au niveau de sites à l'intérieur de FREE2 comprenant la séquence nucléotidique présentée dans la SEQ ID NO: 17 ou une homologue de celle-ci définie comme ayant une identité de séquence nucléotidique d'au moins 80 % avec la SEQ ID NO : 17 ou qui s'hybride à la SEQ ID NO:17 ou à sa forme complémentaire dans des conditions de stringence moyenne en amont du promoteur FMR1 ;
(c) longueur de l'expansion de (CGG)ₙ à l'intérieur du locus génétique FMR lorsqu'elle est considérée en combinaison avec (a) et/ou (b) ;
(2) des moyens pour convertir la valeur de l'indice en un code ; et
(3) des moyens pour stocker le code dans un support lisible par ordinateur.

14. Ordinateur pour évaluer l'association entre l'étendue de la méthylation à l'intérieur du locus FMR, le locus FMR et la progression d'un état pathologique dans lequel l'ordinateur comprend :
(1) un support de stockage de données lisible par machine comprenant un matériau de stockage des données codé au moyen de données lisibles par machine, dans lequel les données lisibles par une machine comprennent des valeurs d'indice associées aux caractéristiques d'un ou plusieurs de ce qui suit :
(a) modification de la méthylation par rapport à un témoin au niveau de sites à l'intérieur de FREE1 comprenant la séquence nucléotidique présentée dans la SEQ ID NO: 16 ou une homologue de celle-ci définie comme ayant une identité de séquence nucléotidique d'au moins 80 % avec la SEQ ID NO : 16 ou qui s'hybride à la SEQ ID NO:16 ou à sa forme complémentaire dans des conditions de stringence moyenne en amont du promoteur FMR1 ;
(b) modification de la méthylation par rapport à un témoin au niveau de sites à l'intérieur de FREE2 comprenant la séquence nucléotidique présentée dans la SEQ ID NO: 17 ou une homologue de celle-ci définie comme ayant une identité de séquence nucléotidique d'au moins 80 % avec la SEQ ID NO : 17 ou qui s'hybride à la SEQ ID NO:17 ou à sa forme complémentaire dans des conditions de stringence moyenne en amont du promoteur FMR1 ;
(c) longueur de l'expansion de (CGG)ₙ à l'intérieur du locus génétique FMR lorsqu'elle est considérée en combinaison avec (a) et/ou (b) ;
(2) des moyens pour convertir la valeur de l'indice en un code ; et
(3) des moyens pour stocker le code dans un support lisible par ordinateur.

15. Procédé *in vitro* d'identification d'un profil épigénétique dans une population de sujets indicatif d'un état pathologique associé aux gènes FMR1, FMR4 et/ou ASFMR1, ledit procédé comprenant la recherche d'une modification par rapport à un témoin chez un nombre statistiquement significatif de sujets de l'étendue de la méthylation à l'intérieur d'une région du locus génétique FMR sélectionnée parmi les régions définies dans la revendication 1, dans lequel une modification de l'étendue de la méthylation est indicative de la présence de l'état pathologique ou d'une propension à développer celui-ci dans cette population.

16. Procédé selon la revendication 15, dans lequel la modification de l'étendue de la méthylation est une augmentation de la méthylation.


### SEQUENCE LISTING

<110> Murdoch Childrens Research Institute Godler , David (US only)
<120> Assay for determining epigenetic profiles of markers of Fragile X alleles
<130> 42.5.110334
<140> 10743317.9
   <141> 2010-02-17
<150> AU2009900668
   <151> 2009-02-17
<150> AU2009901041
   <151> 2009-03-11
<160> 30
<170> PatentIn version 3.5
<210> 1
   <211> 30
   <212> DNA
   <213> Artificial sequence
<220>
   <223> CGGn amplification primer
<400> 1
   gctcagctcc gtttcggttt cacttccggt 30
<210> 2
   <211> 30
   <212> DNA
   <213> Artificial sequence
<220>
   <223> CGGn amplification primer
<400> 2
   agccccgcac ttccaccacc agctcctcca 30
<210> 3
   <211> 30
   <212> DNA
   <213> Artificial sequence
<220>
   <223> Forward primer Amplicon 1
<400> 3
   tgtttatttt tgtagaggtg tatttagtgg 30
<210> 4
   <211> 28
   <212> DNA
   <213> Artificial sequence
<220>
   <223> Reverse primer Amplicon 1
<400> 4
   cttctatcta atccttcacc cctattct 28
<210> 5
   <211> 24
   <212> DNA
   <213> Artificial sequence
<220>
   <223> Forward primer Amplicon 2
<400> 5
   tttaggttat ttgaagagag aggg 24
<210> 6
   <211> 25
   <212> DNA
   <213> Artificial sequence
<220>
   <223> Reverse primer Amplicon 2
<400> 6
   ctccatcttc tcttcaaccc tacta 25
<210> 7
   <211> 29
   <212> DNA
   <213> Artificial sequence
<220>
   <223> Forward primer Amplicon 3
<400> 7
   ttttgttagg tattaagttt aatgttggt 29
<210> 8
   <211> 27
   <212> DNA
   <213> Artificial sequence
<220>
   <223> Reverse primer Amplicon 3
<400> 8
   ccttacaacc ctttacattc cactata 27
<210> 9
   <211> 25
   <212> DNA
   <213> Artificial sequence
<220>
   <223> Forward primer Amplicon 4
<400> 9
   aatgtaaagg gttgtaagga ggtgt 25
<210> 10
   <211> 29
   <212> DNA
   <213> Artificial sequence
<220>
   <223> Reverse primer Amplicon 4
<400> 10
   caaccaaaat aacccaaact tttataacc 29
<210> 11
   <211> 24
   <212> DNA
   <213> Artificial sequence
<220>
   <223> Forward primer Amplicon 5
<400> 11
   ttgaagagaa gatggaggag ttgg 24
<210> 12
   <211> 22
   <212> DNA
   <213> Artificial sequence
<220>
   <223> Reverse primer Amplicon 5
<400> 12
   aaaaaaactt ccaacaaacc cc 22
<210> 13
   <211> 17
   <212> DNA
   <213> Artificial sequence
<220>
   <223> Forward primer FMR4-5'
<400> 13
   ccgcggaatc ccagaga 17
<210> 14
   <211> 19
   <212> DNA
   <213> Artificial sequence
<220>
   <223> Reverse primer FMR4-5'
<400> 14
   cagtggcgtg ggaaatcaa 19
<210> 15
   <211> 17
   <212> DNA
   <213> Artificial sequence
<220>
   <223> probe FMR4-5'
<400> 15
   tgggataacc ggatgca 17
<210> 16
   <211> 220
   <212> DNA
   <213> Artificial sequence
<220>
   <223> FREE1
<400> 16
   agtcttcttc tgtctaatcc ttcaccccta ttctcgcctt ccactccacc tcccgctcag 60
   tcagactgcg ctactttgaa ccggaccaaa ccaaaccaaa ccaaaccaaa ccaaaccaga 120
   ccagacaccc cctcccgcgg aatcccagag aggccgaact gggataaccg gatgcatttg 180
   atttcccacg ccactgagtg cacctctgca gaaatgggcg 220
<210> 17
   <211> 242
   <212> DNA
   <213> Artificial sequence
<220>
   <223> FREE2
<400> 17
   ctgaagagaa gatggaggag ctggtggtgg aagtgcgggg ctccaatggc gctttctaca 60
   aggtacttgg ctctagggca ggccccatct tcgcccttcc ttccctccct tttcttcttg 120
   gtgtcggcgg gaggcaggcc cggggccctc ttcccgagca ccgcgcctgg gtgccagggc 180
   acgctcggcg ggatgttgtt gggagggaag gactggactt ggggcctgtt ggaagcccct 240
   ct 242
<210> 18
   <211> 1679
   <212> DNA
   <213> Artificial sequence
<220>
   <223> FMR1
<400> 18
   cacctacttt gctaggtact aagttcaatg ctggcagtcg tttcttcttt ttttttcttt 60
   tctgttttgc tcaccgattt ctcgttagca cttagcacag tgtctggcac acgatagatg 120
   ctccgtcaac ttctcagttg gataccagca tcccgaaggg aacatggatt aaggcagcta 180
   taagcacggt gtaaaaacag gaataagaaa aagttgaggt ttgtttcaca gtggaatgta 240
   aagggttgca aggaggtgca tcggcccctg tggacaggac gcatgactgc tacacacgtg 300
   ttcaccccac cctctggcac agggtgcaca tacagtaggg gcagaaatga acctcaagtg 360
   cttaacacaa tttttaaaaa atatatagtc aagtgaaagt atgaaaatga gttgaggaaa 420
   ggcgagtacg tgggtcaaag ctgggtctga ggaaaggctc acattttgag atcccgactc 480
   aatccatgtc ccttaaaggg cacagggtgt ctccacaggg ccgcccaaaa tctggtgaga 540
   gagggcgtag acgcctcacc ttctgcctct acgggtcaca aaagcctggg tcaccctggt 600
   tgccactgtt cctagttcaa agtcttcttc tgtctaatcc ttcaccccta ttctcgcctt 660
   ccactccacc tcccgctcag tcagactgcg ctactttgaa ccggaccaaa ccaaaccaaa 720
   ccaaaccaaa ccaaaccaga ccagacaccc cctcccgcgg aatcccagag aggccgaact 780
   gggataaccg gatgcatttg atttcccacg ccactgagtg cacctctgca gaaatgggcg 840
   ttctggccct cgcgaggcag tgcgacctgt caccgccctt cagccttccc gccctccacc 900
   aagcccgcgc acgcccggcc cgcgcgtctg tctttcgacc cggcaccccg gccggttccc 960
   agcagcgcgc atgcgcgcgc tcccaggcca cttgaagaga gagggcgggg ccgaggggct 1020
   gagcccgcgg ggggagggaa cagcgttgat cacgtgacgt ggtttcgtgt ttacacccgc 1080
   agcgggccgg gggttcggcc tcagtcaggc gctcagctcc gtttcggttt cacttccggt 1140
   ggagggccgc ctctgagcgg gcggcgggcc gacggcgagc gcgggcggcg gcggtgacgg 1200
   aggcgccgct gccagggggc gtgcggcagc gcggcggcgg cggcggcggc ggcggcggcg 1260
   gaggcggcgg cggcggcggc ggcggcggcg gctgggcctc gagcgcccgc agcccacctc 1320
   tcgggggcgg gctcccggcg ctagcagggc tgaagagaag atggaggagc tggtggtgga 1380
   agtgcggggc tccaatggcg ctttctacaa ggtacttggc tctagggcag gccccatctt 1440
   cgcccttcct tccctccctt ttcttcttgg tgtcggcggg aggcaggccc ggggccctct 1500
   tcccgagcac cgcgcctggg tgccagggca cgctcggcgg gatgttgttg ggagggaagg 1560
   actggacttg gggcctgttg gaagcccctc tccgactccg agaggcccta gcgcctatcg 1620
   aaatgagaga ccagcgagga gagggttctc tttcggcgcc gagccccgcc ggggtgagc 1679
<210> 19
   <211> 220
   <212> DNA
   <213> Artificial sequence
<220>
   <223> Amplicon 1
<400> 19
   agtcttcttc tgtctaatcc ttcaccccta ttctcgcctt ccactccacc tcccgctcag 60
   tcagactgcg ctactttgaa ccggaccaaa ccaaaccaaa ccaaaccaaa ccaaaccaga 120
   ccagacaccc cctcccgcgg aatcccagag aggccgaact gggataaccg gatgcatttg 180
   atttcccacg ccactgagtg cacctctgca gaaatgggcg 220
<210> 20
   <211> 389
   <212> DNA
   <213> Artificial sequence
<220>
   <223> Amplicon 2
<400> 20
   tcccaggcca cttgaagaga gagggcgggg ccgaggggct gagcccgcgg ggggagggaa 60
   cagcgttgat cacgtgacgt ggtttcgtgt ttacacccgc agcgggccgg gggttcggcc 120
   tcagtcaggc gctcagctcc gtttcggttt cacttccggt ggagggccgc ctctgagcgg 180
   gcggcgggcc gacggcgagc gcgggcggcg gcggtgacgg aggcgccgct gccagggggc 240
   gtgcggcagc gcggcggcgg cggcggcggc ggcggcggcg gaggcggcgg cggcggcggc 300
   ggcggcggcg gctgggcctc gagcgcccgc agcccacctc tcgggggcgg gctcccggcg 360
   ctagcagggc tgaagagaag atggaggag 389
<210> 21
   <211> 260
   <212> DNA
   <213> Artificial sequence
<220>
   <223> Amplicon 3
<400> 21
   cacctacttt gctaggtact aagttcaatg ctggcagtcg tttcttcttt ttttttcttt 60
   tctgttttgc tcaccgattt ctcgttagca cttagcacag tgtctggcac acgatagatg 120
   ctccgtcaac ttctcagttg gataccagca tcccgaaggg aacatggatt aaggcagcta 180
   taagcacggt gtaaaaacag gaataagaaa aagttgaggt ttgtttcaca gtggaatgta 240
   aagggttgca aggaggtgca 260
<210> 22
   <211> 368
   <212> DNA
   <213> Artificial sequence
<220>
   <223> Amplicon 4
<400> 22
   aatgtaaagg gttgcaagga ggtgcatcgg cccctgtgga caggacgcat gactgctaca 60
   cacgtgttca ccccaccctc tggcacaggg tgcacataca gtaggggcag aaatgaacct 120
   caagtgctta acacaatttt taaaaaatat atagtcaagt gaaagtatga aaatgagttg 180
   aggaaaggcg agtacgtggg tcaaagctgg gtctgaggaa aggctcacat tttgagatcc 240
   cgactcaatc catgtccctt aaagggcaca gggtgtctcc acagggccgc ccaaaatctg 300
   gtgagagagg gcgtagacgc ctcaccttct gcctctacgg gtcacaaaag cctgggtcac 360
   cctggttg 368
<210> 23
   <211> 242
   <212> DNA
   <213> Artificial sequence
<220>
   <223> Amplicon 5
<400> 23
   ctgaagagaa gatggaggag ctggtggtgg aagtgcgggg ctccaatggc gctttctaca 60
   aggtacttgg ctctagggca ggccccatct tcgcccttcc ttccctccct tttcttcttg 120
   gtgtcggcgg gaggcaggcc cggggccctc ttcccgagca ccgcgcctgg gtgccagggc 180
   acgctcggcg ggatgttgtt gggagggaag gactggactt ggggcctgtt ggaagcccct 240
   ct 242
<210> 24
   <211> 214
   <212> DNA
   <213> Artificial sequence
<220>
   <223> Amplicon 1 after C/T conversion
<210> 25
   <211> 387
   <212> DNA
   <213> Artificial sequence
<220>
   <223> Amplicon 2 after C/T conversion
<400> 25
   tttaggttat ttgaagagag agggcggggt cgaggggttg agttcgcggg gggagggaat 60
   agcgttgatt acgtgacgtg gttttagtgt ttatattcgt agcgggtcgg gggttcggtt 120
   ttagttaggc gtttagtttc gtttcggttt tattttcggt ggagggtcgt ttttgagcgg 180
   gcggcgggtc gacggcgagc gcgggcggcg gcggtgacgg aggcgtcgtt gttagggggc 240
   gtgcggtagc gcggcggcgg cggcggcggc ggcggcggcg gaggcggcgg cggcggcggc 300
   ggcggcggcg gttgggtttc gagcgttcgt agtttatttt tcgggggcgg gttttcggcg 360
   ttagtagggt tgaagagaag atggagg 387
<210> 26
   <211> 248
   <212> DNA
   <213> Artificial sequence
<220>
   <223> Amplicon 3 after C/T conversion
<400> 26
   ttgttaggta ttaagtttaa tgttggtagt cgtttttttt tttttttttt tttttgtttt 60
   gtttatcgat ttttcgttag tatttagtat agtgtttggt atacgataga tgtttcgtta 120
   attttttagt tggatattag tatttcgaag ggaatatgga ttaaggtagt tataagtacg 180
   gtgtaaaaat aggaataaga aaaagttgag gtttgtttta tagtggaatg taaagggttg 240
   taaggagg 248
<210> 27
   <211> 368
   <212> DNA
   <213> Artificial sequence
<220>
   <223> Amplicon 4 after C/T conversion
<400> 27
   aatgtaaagg gttgcaagga ggtgcatcgg cccctgtgga caggacgcat gactgctaca 60
   cacgtgttca ccccaccctc tggcacaggg tgcacataca gtaggggcag aaatgaacct 120
   caagtgctta acacaatttt taaaaaatat atagtcaagt gaaagtatga aaatgagttg 180
   aggaaaggcg agtacgtggg tcaaagctgg gtctgaggaa aggctcacat tttgagatcc 240
   cgactcaatc catgtccctt aaagggcaca gggtgtctcc acagggccgc ccaaaatctg 300
   gtgagagagg gcgtagacgc ctcaccttct gcctctacgg gtcacaaaag cctgggtcac 360
   cctggttg 368
<210> 28
   <211> 0
   <212> DNA
   <213> Artificial sequence
<220>
   <223> Amplicon 5 after C/T conversion
<400> 28
   000
<210> 29
   <211> 31
   <212> DNA
   <213> Artificial sequence
<220>
   <223> T7 promoter for Amplicons 1-5
<400> 29
   cagtaatacg actcactata gggagaaggc t 31
<210> 30
   <211> 10
   <212> DNA
   <213> Artificial sequence
<220>
   <223> 5' of primer for Amplicons 1-5
<400> 30
   aggaagagag 10